# EUROPEAN PATENT APPLICATION

(11) **EP 4 800 017 A1**
(43) Date of publication of application: **02.09.2026**
(21) Application number: 24882817.0
(22) Date of filing: 23.10.2024
(51) Int. Cl.: C07D 487/08, A61K 9/51, A61K 31/7088, A61K 48/00, C07D 231/12

(54) **IONIZABLE LIPID AND USE THEREOF**

(30) Priority: 24.10.2023 KR 20230143205; 25.10.2023 KR 20230143867
(71) Applicant: GREEN CROSS CORPORATION, Yongin-si, Gyeonggi-do 16924 (KR)
(72) Inventor: YOO, Hyun Jung, Yongin-si, Gyeonggi-do 16924 (KR); SON, Ji Yeon, Yongin-si, Gyeonggi-do 16924 (KR); BAN, Jae Young, Yongin-si, Gyeonggi-do 16924 (KR); KANG, Sun Hee, Yongin-si, Gyeonggi-do 16924 (KR); JEONG, Jae Uk, Yongin-si, Gyeonggi-do 16924 (KR); KIM, Jung Hyun, Yongin-si, Gyeonggi-do 16924 (KR); KIM, Jee Won, Yongin-si, Gyeonggi-do 16924 (KR); PARK, Eun Young, Yongin-si, Gyeonggi-do 16924 (KR)
(74) Representative: HGF
(86) International application number: PCT/KR2024/016193
(87) International publication number: WO 2025/089790

(57) **Abstract**

The present invention relates to a novel ionizable lipid compound represented by Formula (I), or a salt thereof, and lipid nanoparticles comprising same. The lipid nanoparticles comprising the novel ionizable lipid compound, according to the present invention, have excellent nucleic acid encapsulation efficiency and high nucleic acid cell delivery efficiency.

## Description

### Technical Field

The present invention relates to a novel ionizable lipid or a salt thereof, a lipid nanoparticle including the ionizable lipid or the salt thereof, and a composition including an ionic drug and the ionizable lipid or salt thereof or the lipid nanoparticle.

### Background Art

With the development of biotechnology, studies for the prevention or treatment of diseases by delivering nucleic acids such as RNA interference therapeutics, antisense oligonucleotides (ASO), short interfering RNA (siRNA), aptamers, messenger RNA (mRNA), self-amplifying RNA (SAM), and circular RNA into the body have been actively conducted.

Meanwhile, nucleic acids have a disadvantage in that they are easily decomposed in the blood by the enzymes in the body such as RNase and nuclease. In addition, among nucleic acids, mRNA with a length of 500 nucleotides or more has a large molecular weight of 200 KDa or more, thus making it difficult to deliver nucleic acids to target cells in the body, and the negatively charged characteristic of nucleic acids is a factor that hinders the delivery of nucleic acids to target cells in the body. Accordingly, there has been a growing importance of lipid nanoparticles (LNPs) as a drug delivery vehicle that facilitate delivery of nucleic acids to target cells while preventing degradation of nucleic acids in the body. In fact, LNPs were also used in the recent development of mRNA-based Covid-19 vaccines (Spikevax^{™}, Comirnaty^{™}).

LNPs are generally known to be composed of an ionizable lipid, a helper lipid (*e.g.,* phospholipid), cholesterol, and PEG-lipids.

The ionizable lipids that make up LNPs are typically composed of an ionizable head, a connecting part (linker), and a terminal end consisting of a hydrocarbon chain, and they are known to play an essential role in determining the efficiency of nucleic acid delivery by electrostatically binding to nucleic acids, encapsulating the nucleic acids, and enabling the escape of nucleic acids from endosomes within cells. Additionally, cholesterol maintains LNP particles hard and stable, and PEG-lipids play a role in preventing aggregation between LNP particles and ensuring structural stability. Moreover, helper lipids *(e.g.,* phospholipids) serve to maintain the lipid bilayer structure of the LNP particles.

### [Prior Art Document]

(Patent Document 1) U.S. Patent Application Publication No. US 2020/0140378 A1

### Disclosure of Invention

### Technical Problem

An object of the present invention is to provide a novel ionizable lipid compound or a salt thereof, which has the characteristics of excellent nucleic acid delivery efficiency into cells and nucleic acid encapsulation efficiency while minimizing degradation of nucleic acids, a lipid nanoparticle including the same, and a composition for drug delivery.

### Solution to Problem

The present inventors have synthesized and prepared a novel ionizable lipid compound by introducing a diazabicyclo structure to the head portion of the ionizable lipid, adding an ester group or branched ester group to the terminal end hydrocarbon chain of the ionizable lipid, or additionally, by repeatedly substituting the ester group with another group, and have found that LNPs including the novel ionizable lipid have excellent nucleic acid encapsulation efficiency and nucleic acid cell delivery efficiency, thereby completing the present invention.

The novel ionizable lipid compound or a salt thereof of the present invention by introducing a diazabicyclo structure, in particular by introducing the 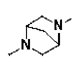 structure, to the head portion of the ionizable lipid, were shown to be similar or superior with Z-average diameter, the homogeneity of particle size (*e.g*., a polydispersity index (PDI)), encapsulation efficiency of a nucleic acid (*e.g*., mRNA), efficacy of nucleic acid delivery into cells, *etc*., when compared to other ionizable lipid compounds with a similar structure in which the head portion of the ionizable lipid is piperazine, while including an ester group or having a branched ester structure in the terminal end hydrocarbon chain of the ionizable lipid.

In an embodiment of the present invention, the present invention provides a compound represented by Formula (I) or Formula (IV) or a salt thereof.

In another embodiment of the present invention, the present invention provides a lipid nanoparticle, which includes a compound represented by Formula (I) or Formula (IV) or a salt thereof, a helper lipid, cholesterol, and a PEG lipid.

In still another embodiment of the present invention, the present invention provides a composition which includes an ionic drug and (i) the compound represented by Formula (I) or Formula (IV) above or a salt thereof, or a composition which includes an ionic drug and (ii) the lipid nanoparticles.

### Advantageous Effects of Invention

The novel ionizable lipid compound or a salt thereof of the present invention has excellent efficiency in delivering nucleic acids to cells and stability in the body, and thus, can contribute to improving the *in vivo* stability and therapeutic efficacy of nucleic acid therapeutics. The novel ionizable lipid compound of the present invention or a salt thereof has a nucleic acid encapsulation efficiency of 93% or higher and excellent efficacy of nucleic acid delivery into cells.

### Brief Description of Drawings

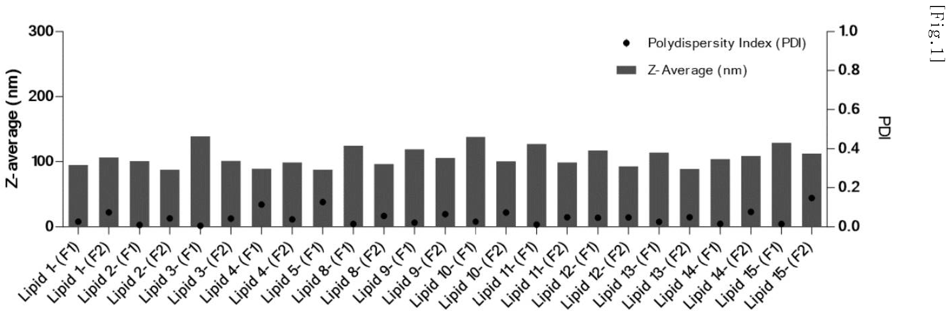

Fig. 1 is a graph illustrating the z-average diameter and the polydispersity index (PDI) of F1-type LNPs including any one of Lipid 1 to Lipid 5 and Lipid 8 to Lipid 15 (*i.e*., Lipid 1-(F1) to Lipid 5-(F1) and Lipid 8-(F1) to Lipid 15-(F1)) and F2-type LNPs including any one of Lipid 1 to Lipid 4, and Lipid 8 to Lipid 15 (*i.e*., Lipid 1-(F2) to Lipid 4-(F2) and Lipid 8-(F2) to Lipid 15-(F2)), which is an embodiment of the ionizable lipid compound of the present invention.

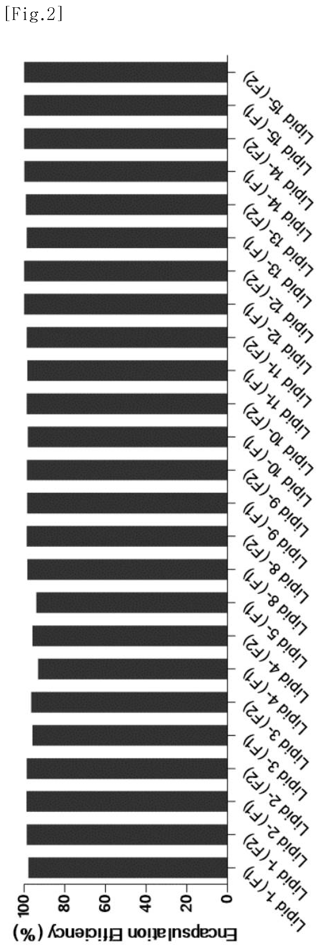

Fig. 2 is a graph illustrating the encapsulation efficiency of F1-type LNPs including any one of Lipid 1 to Lipid 5 and Lipid 8 to Lipid 15 (*i.e*., Lipid 1-(F1) to Lipid 5-(F1), and Lipid 8-(F1) to Lipid 15-(F1)) and F2-type LNPs including any one of Lipid 1 to Lipid 4 and Lipid 8 to Lipid 15 (*i.e*., Lipid 1-(F2) to Lipid 4-(F2), and Lipid 8-(F2) to Lipid 15-(F2)), which is an embodiment of the ionizable lipid compound of the present invention.

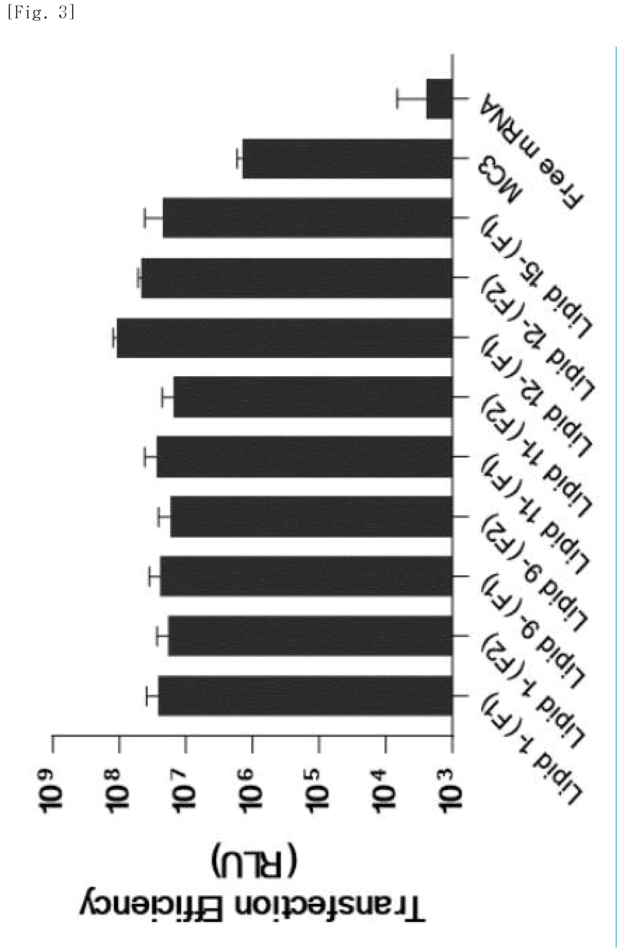

Fig. 3 is a graph illustrating the nucleic acid delivery efficiency into cells of the experimental groups (LNPs including Lipid 1-(F1), Lipid 1-(F2), Lipid 9-(F1), Lipid 9-(F2), Lipid 11-(F1), Lipid 11-(F2), Lipid 12-(F1), Lipid 12-(F2), or Lipid 15-(F1)), a positive control group (MC3 LNP), and a negative control group (free mRNA).

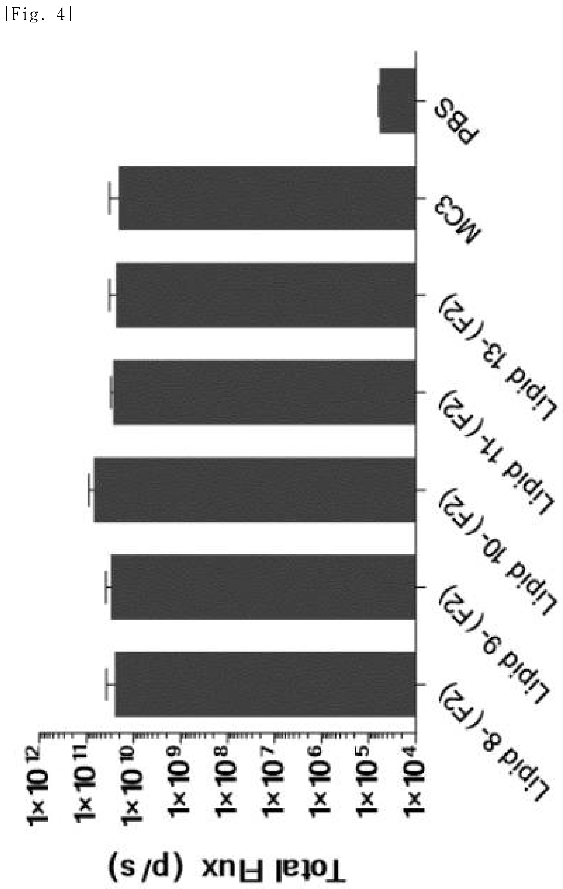

Fig. 4 is a graph illustrating the expression rate of luminescent firefly luciferase protein in the liver region of mice when the experimental groups (LNPs including Lipid 8-(F2), Lipid 9-(F2), Lipid 10-(F2), Lipid 11-(F2), or Lipid 13-(F2)) and the positive control group (MC3 LNP) were mixed with luminescent firefly luciferase mRNA nucleic acid and administered into the tail vein of mice or when only PBS (a negative control group) was administered into the tail vein of mice.

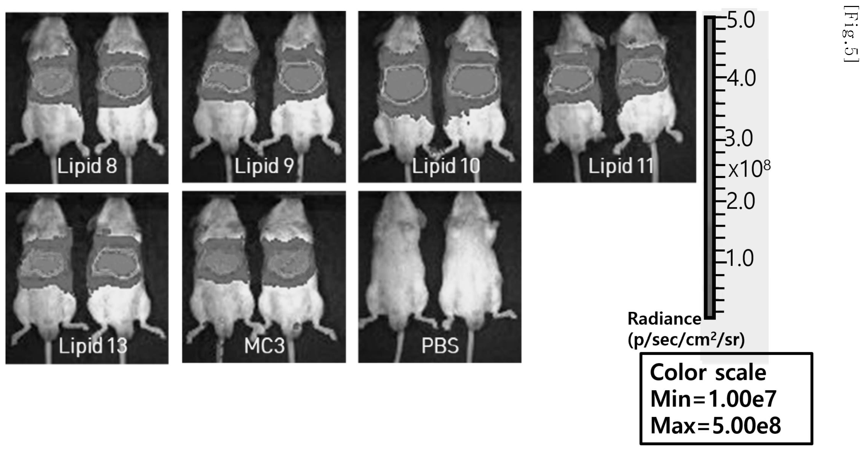

Fig. 5 shows images illustrating the presence/absence of expression of luminescent firefly luciferase protein in the liver region of mice when the experimental groups (LNPs including Lipid 8-(F2), Lipid 9-(F2), Lipid 10-(F2), Lipid 11-(F2), or Lipid 13-(F2)) and the control group (MC3 LNP) were mixed with luminescent firefly luciferase mRNA nucleic acid and administered into the tail vein of mice or when only PBS (a negative control group) was administered into the tail vein of mice.

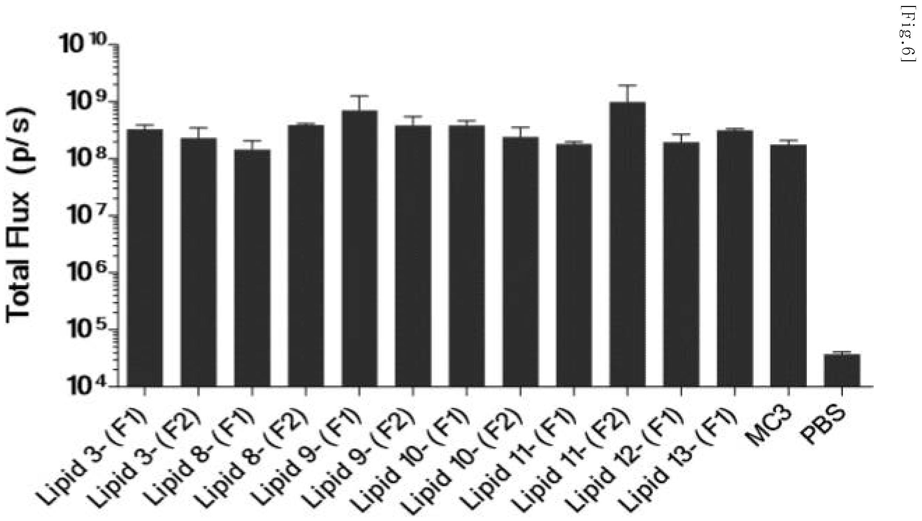

Fig. 6 is a graph illustrating the protein expression rate of luminescent firefly luciferase mRNA delivered to the muscle of mice when the experimental groups (LNPs including Lipid 3-(F1), Lipid 3-(F2), Lipid 8-(F1), Lipid 8-(F2), Lipid 9-(F1), Lipid 9-(F2), Lipid 10-(F1), Lipid 10-(F2), Lipid 11-(F1), Lipid 11-(F2), Lipid 12-(F1), or Lipid 13-(F1)) and the positive control group (MC3 LNP) were mixed with luminescent firefly luciferase mRNA nucleic acid and administered into the thigh muscle of mice or when only PBS (a negative control group) was administered into the thigh muscle of mice.

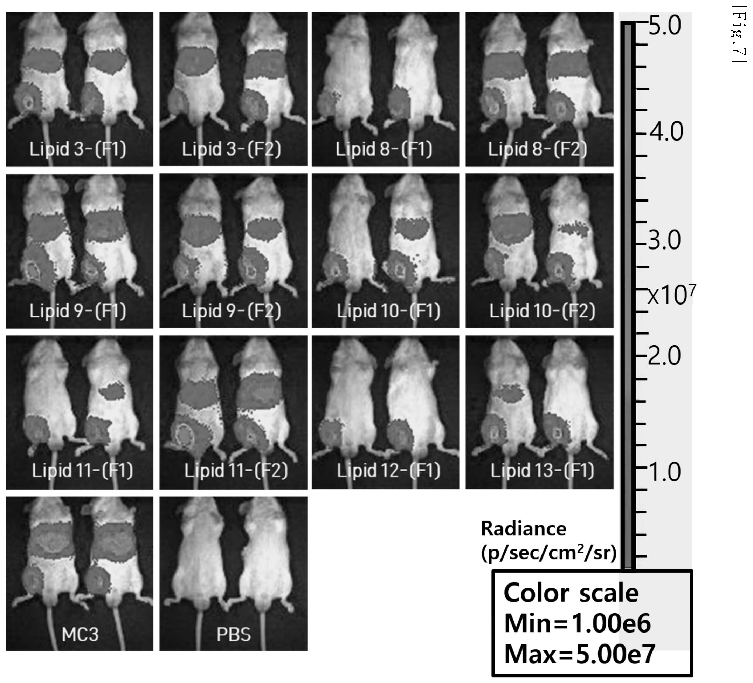

Fig. 7 shows images illustrating the presence/absence of expression of luminescent firefly luciferase protein in the muscle of mice when the experimental groups (LNPs including Lipid 3-(F1), Lipid 3-(F2), Lipid 8-(F1), Lipid 8-(F2), Lipid 9-(F1), Lipid 9-(F2), Lipid 10-(F1), Lipid 10-(F2), Lipid 11-(F1), Lipid 11-(F2), Lipid 12-(F1), or Lipid 13-(F1)) and the positive control group (MC3 LNP) were mixed with luminescent firefly luciferase mRNA nucleic acid and administered into the thigh muscle of mice or when only PBS (a negative control group) was administered into the thigh muscle of mice.

### Best Mode for Carrying out the Invention

### Definition

As used herein, the term "aliphatic amine" refers to a linear or branched hydrocarbon functional group having an amine terminal end, and it may have an aliphatic group having one or more unsaturated regions. The aliphatic amine, which is a functional group including one or more amine groups and one or more aliphatic groups, includes triethylamine, tripropylamine, tributylamine, diisopropylamine, triisopropylamine, triisobutylamine, *N,N*-diisopropylethylamine, stearylamine, ethylenediamine, propylenediamine, hexamethylenediamine, di(6-aminohexyl)amine, decylamine, undecylamine, dodecylamine, tridecylamine, tetradecylamine, hexadecylamine, octadecylamine, oleylamine, docosylamine, *etc*., but is not limited thereto.

As used herein, the term "alkyl" refers to a functional group derived from a linear chain or branched hydrocarbon, and it may include methyl, ethyl, *N*-propyl, *i*-propyl, *N-*butyl, *i*-butyl, *t*-butyl, *N*-pentyl, *N*-hexyl, *etc*., but is not limited thereto.

Additionally, as used herein, the term "cycloalkyl" refers to a saturated carbocyclic group of carbon atoms having not only a single ring (*e.g*., cyclohexyl) but also multiple fused rings (*e.g*., norbornyl, adamantyl), and includes cyclopropyl, cyclopentyl, cyclohexyl, norbornyl, adamantyl, *etc*., but is not limited thereto.

Additionally, as used herein, the term "heterocycloalkyl" refers to a mono- or polycyclic ring including necessarily one or more, preferably 1 to 4 of heteroatoms of O, N, or S, and does not include an aromatic ring. For example, it includes pyrrolidine, imidazoline, imidazolidine, pyrazoline, pyrazolidine, piperidine, morpholine, piperazine, tetrahydropyridinyl, *etc*.

As used herein, the term "lipid nanoparticle (LNP)" refers to a structure in which a drug (*e.g*., a nucleic acid-based drug) is mixed with a lipid at room temperature to form a uniform phase, which is then dispersed in an aqueous solution so that the drug exists as a solid solution between lipid crystals. The lipid nanoparticle has high bioavailability and affinity because it employs substances that exist in the body, such as phospholipids, lipids, and cholesterol, and it is a particulate drug delivery vehicle that enables drug release and control, and has high stability against decomposition by enzymes, *etc*. Additionally, lipid nanoparticles can encapsulate RNA by being positively (+) charged at an acidic pH, and are neutrally charged at a physiological pH, thereby minimizing toxicity.

### Ionizable lipid compound

The ionizable lipid compound of the present invention may be a compound represented by Formula (I) or Formula (IV) described below, or a salt thereof.
[1] The compound of the present invention may be a compound represented by the following Formula (I) or a salt thereof:

   L1-L2-L3-L4-L5 (I)

   L1 and L5 above are each independently a group represented by following Formula (II), 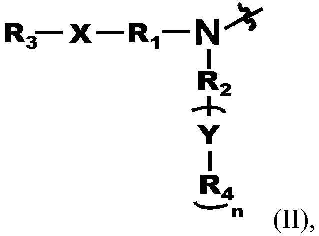
   L2 is a C₁₋₁₂ linear alkyl, which is substituted or unsubstituted with a hydroxy group, in which when L2 is a C₁₋₁₂ linear alkyl substituted with a hydroxy group, Formula (I) does not include L1,
   L4 is a C₁₋₁₂ linear alkyl,
   L3 is a 6-membered heterocyclic amine or aliphatic amine, in which the 6-membered heterocyclic amine or aliphatic amine is substituted with at least one selected from the group consisting of C₁₋₆ alkyl, an amino group, 3- to 6-membered saturated or unsaturated carbocyclyl, 3- to 6-membered saturated or unsaturated carbocycloalkyl, 3-to 6-membered saturated or unsaturated heterocyclyl, and 3- to 6-membered saturated or unsaturated heterocycloalkyl, and n is an integer of 0 or 1,
   X and Y are, each independently, -Rx'-C(=O)-O-, -Rx'-C(=O)-, -Rx'-NHC(=O)-, - C(=O)NH-Rx'-, -Rx'-O-, or -Rx'-NH-,
   in which Rx' is hydrogen, linear C₁-₂₅ alkyl, or branched C₃-₂₅ alkyl,
   R₁ and R₂, which are each independently linear C₁-₃₀ alkyl or branched C₃-₅₀ alkyl, are the same as or different from each other,
   R₃ and R₄, which are each independently linear C₁-₃₀ alkyl, linear C₁-₃₀ alkylene, branched C₃-₅₀ alkyl or C₄₋₄₀ alkoxy, are the same as or different from each other, and the linear C₁-₃₀ alkyl, linear C₁-₃₀ alkylene, branched C₃-₅₀ alkyl, or C₄₋₄₀ alkoxy is substituted or unsubstituted with at least one selected from the group consisting of C₁₋₁₂ alkyl, Rx"-C(=O)-O-, Rx"-O-C(=O)-, and amine,
   Rx" is hydrogen, linear C₁-₂₅ alkyl, branched C₃-₂₅ alkyl, 3- to 6-membered saturated or unsaturated carbocyclyl, 3- to 6-membered saturated or unsaturated carbocycloalkyl, 3- to 6-membered saturated or unsaturated heterocyclyl, or 3- to 6-membered saturated or unsaturated heterocycloalkyl,
   the wavy line represents a bonding position,
   the heterocyclic amine is a cyclic amine group including one or more heteroatoms selected from N, S, and O,
   the carbocyclyl is a cyclic group consisting of carbon and hydrogen atoms,
   the heterocyclyl is a cyclic group including one or more heteroatoms selected from N, S, and O,
   the carbocycloalkyl is a carbocyclyl-linear C₁-₆ alkyl group or carbocyclyl-branched C₃-₆ alkyl group, and
   the heterocycloalkyl is a heterocyclyl-linear C₁-₆ alkyl group or heterocyclyl-branched C₃-₆ alkyl group,
   in which L3 is not piperazine.
[2] In [1] above, L3 may be a 6-membered heterocyclic amine or 6-membered heterocyclic amine including two N atoms, and may have a diazabicyclo structure. In particular, L3 of Formula (I) may have a 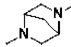 structure.
[3] In [1] and [2] above, L1 and L5 of Formula (I) above may each independently be a compound represented by the following Formula (III) or a salt thereof: 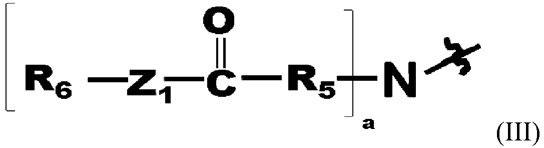 in which R₅ is linear C₁-₃₀ alkyl,
   Z₁ is -(Rx^{a})ₖ-O- or -(Rx^{a})ₖ-NH-, Rx^{a} is linear C₁-₂₅ alkyl, the k is an integer of 0 or 1,
   R₆ is linear C₁-₃₀ alkyl, linear C₁-₃₀ alkylene, branched C₃-₅₀ alkyl, or C₄₋₄₀ alkoxy, in which the linear C₁-₃₀ alkyl, linear C₁-₃₀ alkylene, branched C₃-₅₀ alkyl, or C₄₋₄₀ alkoxy is substituted or unsubstituted with at least one selected from the group consisting of C₁₋₁₂ alkyl, Rx^{b}-C(=O)-O-, Rx^{b}-O-C(=O)-, and amine, Rx^{b} is hydrogen, linear C₁-₂₅ alkyl, branched C₃-₂₅ alkyl, 3- to 6-membered saturated or unsaturated carbocyclyl, 3- to 6-membered saturated or unsaturated carbocycloalkyl, 3- to 6-membered saturated or unsaturated heterocyclyl, or 3- to 6-membered saturated or unsaturated heterocycloalkyl, the a is an integer of 1 or 2, the carbocyclyl is a cyclic group consisting of carbon and hydrogen atoms, the heterocyclyl is a cyclic group including one or more heteroatoms selected from N, S, and O, the carbocycloalkyl is a carbocyclyl-linear C₁-₆ alkyl group or carbocyclyl-branched C₃-₆ alkyl group, and the heterocycloalkyl is a heterocyclyl-linear C₁-₆ alkyl group or heterocyclyl-branched C₃-₆ alkyl group.
[4] In any one of [1] to [3] above, R₅ may be linear C₂-₃₀ alkyl, linear C₂-₂₀ alkyl, or linear C₂-₁₀ alkyl, the Z₁ may be -(Rx^{a})ₖ-O-, and the k may be an integer of 0.
[5] In another aspect of the present invention, the compound of the present invention may be a compound represented by the following Formula (IV) or a salt thereof: 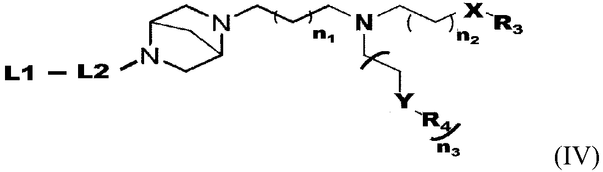 In Formula (IV) above, the definitions of L1, L2, X, Y, R₃, and R₄ are the same as those of L1, L2, X, Y, R₃, and R₄ in Formula (I) above, and the n₁ and n₂ are each an integer of 0 to 12, and the n₃ is an integer of 0 or 1.
[6] In any one of [1] to [5] above, L2 may be C₁₋₆ linear alkyl, or C₁₋₆ linear alkyl substituted with a hydroxy group, and/or L4 may be a C₁₋₆ linear alkyl compound or a salt thereof. Additionally, L2 may be C₁₋₃ linear alkyl, C₃₋₆ linear alkyl, C₁₋₃ linear alkyl substituted with a hydroxy group, or C₃₋₆ linear alkyl substituted with a hydroxy group, and/or L4 may be C₁₋₃ linear alkyl or C₃₋₆ linear alkyl. Additionally, L2 may be C₃₋₄ linear alkyl, or C₃₋₄ linear alkyl substituted with a hydroxy group, and/or L4 may be C₃₋₄ linear alkyl.
[7] In any one of [1] to [6] above, X and Y may each independently be -Rx'-C(=O)-O-, -Rx'-C(=O)-, -Rx'-NHC(=O)-, -C(=O)NH-Rx'-, -Rx'-O-, or -Rx'-NH-, and Rx' may be hydrogen, or linear C₁-₂₅ alkyl. The X and Y may be -Rx'-C(=O)-O-, and Rx' may be linear C₁-₂₅ alkyl.
[8] In any one of [1] to [7] above, R₁ and R₂ may each independently be linear C₁-₃₀ alkyl or linear C₁-₁₅ alkyl, and may be the same as or different from each other.
[9] In any one of [1] to [8] above, R₁ and R₂ may each independently be linear C₁-₁₂ alkyl, and may be the same as or different from each other.
[10] In any one of [1] to [9] above, R₃ and R₄ may each independently be linear C₁-₂₀ alkyl, linear C₁-₂₀ alkylene, or branched C₃-₄₀ alkyl or C₄₋₃₀ alkoxy, and may be unsubstituted or substituted with at least one selected from the group consisting of C₁₋₁₂ alkyl, Rx"-C(=O)-O-, Rx"-O-C(=O)-, and amine.
[11] In any one of [1] to [10] above, L2 may be C₁₋₆ linear alkyl or C₁₋₆ linear alkyl substituted with a hydroxy group, L4 may be C₁₋₆ linear alkyl, the X and Y may each independently be -Rx'-C(=O)-O-, -Rx'-C(=O)-, -Rx'-NHC(=O)-, -C(=O)NH-Rx'-, -Rx'-O-, or -Rx'-NH-, Rx' may be hydrogen or linear C₁-₂₅ alkyl, R₁ and R₂ may each independently be linear C₁-₃₀ alkyl or branched C₃-₅₀ alkyl and may be the same as or different from each other, R₃ and R₄ may each independently be linear C₁-₂₀ alkyl, linear C₁-₂₀ alkylene, or branched C₃-₄₀ alkyl or C₄₋₃₀ alkoxy and may be the same as or different from each other, and the linear C₁-₂₀ alkyl, linear C₁-₂₀ alkylene, and branched C₃-₄₀ alkyl or C₄₋₃₀ alkoxy may be unsubstituted or substituted with at least one selected from the group consisting of C₁₋₁₂ alkyl, Rx"-C(=O)-O-, Rx"-O-C(=O)-, and amine.
[12] In any one of [1] to [11] above, in Formula (I) above, L3 may have a diazabicyclo structure, L2 may be C₁₋₄ linear alkyl or C₁₋₄ linear alkyl substituted with a hydroxy group, L4 may be C₁₋₆ linear alkyl, and X and Y may each independently be - Rx'-C(=O)-O-, -Rx'-C(=O)-, -Rx'-NHC(=O)-, -C(=O)NH-Rx'-, -Rx'-O-, or -Rx'-NH-, Rx' may be hydrogen or linear C₁-₁₂ alkyl, R₁ and R₂ may each independently be linear C₂-₈alkyl and may be the same as or different from each other, R₃ and R₄ may each independently be linear C₁-₂₀ alkyl, linear C₁-₂₀ alkylene, or branched C₃-₄₀ alkyl or C₄₋₃₀ alkoxy and may be the same as or different from each other, and the linear C₁-₂₀ alkyl, linear C₁-₂₀ alkylene, and branched C₃-₄₀ alkyl or C₄₋₃₀ alkoxy may be unsubstituted or substituted with at least one selected from the group consisting of C₁₋₁₂ alkyl, Rx"-C(=O)-O-, Rx"-O-C(=O)-, and amine.

Additionally, L3 may have a diazabicyclo structure, L2 may be C₁₋₄ linear alkyl, L4 may be C₁₋₄ linear alkyl, X and Y may each independently be -Rx'-C(=O)-O-, -Rx'-C(=O)-, -Rx'-NHC(=O)-, -C(=O)NH-Rx'-, -Rx'-O-, or -Rx'-NH-, Rx' may be hydrogen or linear C₁-₁₂ alkyl, R₁ and R₂ may each independently be linear C₂-₈alkyl and may be the same as or different from each other, R₃ and R₄ may each independently be linear C₁-₂₀ alkyl, linear C₁-₂₀ alkylene, branched C₃-₄₀ alkyl or C₄₋₃₀ alkoxy and may be the same as or different from each other, and the linear C₁-₂₀ alkyl, linear C₁-₂₀ alkylene, and branched C₃-₄₀ alkyl or C₄₋₃₀ alkoxy may be unsubstituted or substituted with at least one selected from the group consisting of C₁₋₁₂ alkyl, Rx"-C(=O)-O-, Rx"-O-C(=O)-, and amine.

Furthermore, L3 may have a diazabicyclo structure, L2 may be C₁₋₄ linear alkyl substituted with a hydroxy group, L4 is C₁₋₆ linear alkyl, X and Y may each independently be -Rx'-C(=O)-O-, -Rx'-C(=O)-, -Rx'-NHC(=O)-, -C(=O)NH-Rx'-, -Rx'-O-, or -Rx'-NH-, Rx' may be hydrogen or linear C₁-₁₂ alkyl, R₁ and R₂ may each independently be linear C₂-₈ alkyl and may be the same as or different from each other, R₃ and R₄ may each independently be linear C₁-₂₀ alkyl, linear C₁-₂₀ alkylene, or branched C₃-₄₀ alkyl or C₄₋₃₀ alkoxy and may be the same as or different from each other, and the linear C₁-₂₀alkyl, linear C₁-₂₀ alkylene, and branched C₃-₄₀ alkyl or C₄₋₃₀ alkoxy may be unsubstituted or substituted with at least one selected from the group consisting of C₁₋₁₂ alkyl, Rx"-C(=O)-O-, Rx"-O-C(=O)-, and amine.

In any one of [1] to [12] above, the compound of the present invention may be a compound selected from the following group or a salt thereof:
(1) ((3,3',3",3‴-(((2,5-diazabicyclo[2.2.1]heptane-2,5-diyl)bis(propane-3,1-diyl))bis(azanetriyl)) tetrakis(propanoyl))tetrakis(oxy))tetrakis(butane-4,1-diyl)tetranonanoate;
(2) tetrakis(2-ethylhexyl) 3,3',3",3"'-(((2,5-diazabicyclo[2.2.1]heptane-2,5-diyl)bis(propane-3,1-diyl))bis(azanetriyl))tetrapropionate;
(3) tetrakis(4-(2-cyclohexylacetoxy)butyl) 3,3',3",3‴-(((2,5-diazabicyclo[2.2.1]heptane-2,5-diyl)bis(propane-3,1-diyl))bis(azanetriyl))tetrapropionate;
(4) ((3,3',3",3‴-(((2,5-diazabicyclo[2.2.1]heptane-2,5-diyl)bis(propane-3,1-diyl))bis(azanetriyl))tetrakis(propanoyl))tetrakis(oxy))tetrakis(butane-4,1-diyl)tetraheptanoate;
(5) ((3,3',3",3‴-(((2,5-diazabicyclo[2.2.1]heptane-2,5-diyl)bis(propane-3,1-diyl))bis(azanetriyl)) tetrakis(propanoyl))tetrakis(oxy))tetrakis(ethane-2,1-diyl)tetraheptanoate;
(6) ((3,3',3",3‴-(((2,5-diazabicyclo[2.2.1]heptane-2,5-diyl)bis(propane-3,1-diyl))bis(azanetriyl)) tetrakis(propanoyl))tetrakis(oxy))tetrakis(butane-4,1-diyl)tetratridecanoate;
(7) tetrakis(2-ethylhexyl) 4,4',4",4‴-((3,3',3",3‴-(((2,5-diazabicyclo[2.2.1]heptane-2,5-diyl)bis(propane-3,1-diyl))bis(azanetriyl))tetrakis(propanoyl))tetrakis(oxy))tetrabutyrate;
(8) tetrakis(2-ethylhexyl) 6,6',6",6‴-((3,3',3",3‴-(((2,5-diazabicyclo[2.2.1]heptane-2,5-diyl)bis(propane-3,1-diyl))bis(azanetriyl))tetrakis(propanoyl))tetrakis(oxy))tetrahexanoate;
(9) tetrakis(2-propylhexyl) 4,4',4",4‴-((3,3',3",3‴-(((2,5-diazabicyclo[2.2.1]heptane-2,5-diyl)bis(propane-3,1-diyl))bis(azanetriyl))tetrakis(propanoyl))tetrakis(oxy))tetrabutyrate;
(10) tetrakis(2-ethylpentyl) 4,4',4'',4‴-((3,3',3'',3‴-(((2,5-diazabicyclo[2.2.1]heptane-2,5-diyl)bis(propane-3,1-diyl))bis(azanetriyl))tetrakis(propanoyl))tetrakis(oxy))tetrabutyrate;
(11) tetrakis(2-ethylpentyl) 6,6',6'',6‴-((3,3',3'',3‴-(((2,5-diazabicyclo[2.2.1]heptane-2,5-diyl)bis(propane-3,1-diyl))bis(azanetriyl))tetrakis(propanoyl))tetrakis(oxy))tetrahexanoate;
(12) tetra((Z)-non-2-en-1-yl) 9,9',9'',9‴-(((2,5-diazabicyclo[2.2.1]heptane-2,5-diyl)bis(propane-3,1-diyl))bis(azanetriyl))tetranonanoate;
(13) (((3,3',3'',3‴-(((2,5-diazabicyclo[2.2.1]heptane-2,5-diyl)bis(propane-3,1-diyl))bis(azanetriyl))tetrakis(propanoyl))tetrakis(oxy))tetrakis(methylene))tetrakis(prop ane-2,1,3-triyl)octahexanoate;
(14) di(henicosan-10-yl) 3,3'-(((2,5-diazabicyclo[2.2.1]heptane-2,5-diyl)bis(propane-3,1-diyl))bis(methylazanediyl))dipropionate; and
(15) ((3,3'-((3-(5-(3-hydroxypropyl)-2,5-diazabicyclo[2.2.1]heptane-2-yl)propyl)azanediyl)bis (propanoyl))bis(oxy))bis(hexane-6,1-diyl)bis(2-hexyldecanoate).

The ionizable lipid compound of the present invention may have an asymmetric center, a chiral axis, and a chiral plane, and may appear as a racemate, a racemic mixture, and an individual diastereomer, and in particular, all possible isomers including optical isomers and mixtures thereof are included in the present invention.

The compound, such as a compound of Formula (I) or Formula (IV), or a salt thereof may be prepared by a method illustrated in the following Reaction Scheme 1, 2, or 3, but is not limited to those prepared by such methods.

Reaction Schemes 1 to 3 illustrate the method of preparing representative compounds according to the present invention in each preparation step. Various compounds of the present invention may be prepared by changing the reagents and solvents used in the preparation steps below or by changing the reaction sequence. 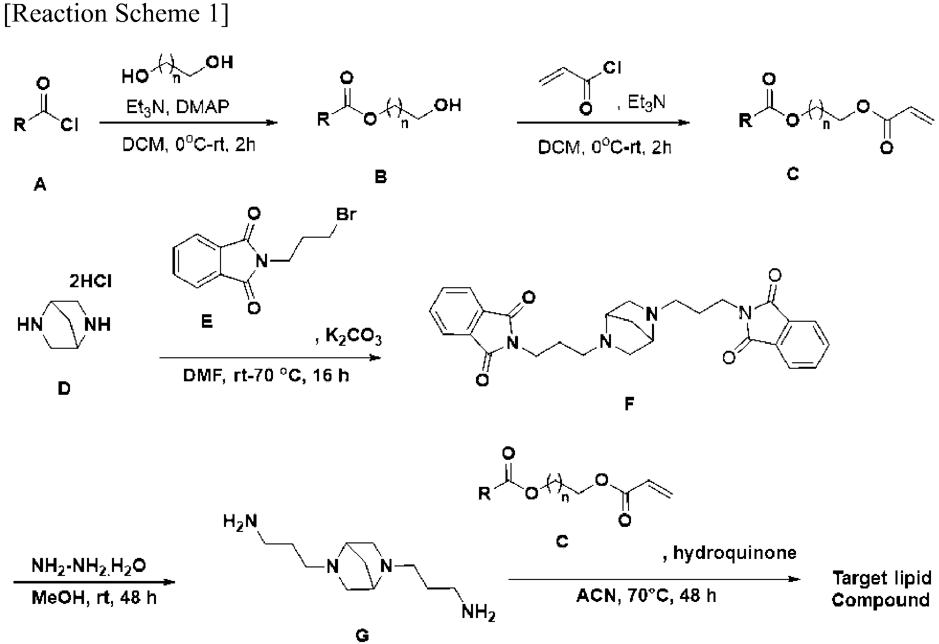
R: linear alkyl, branched alkyl, or oxycarbonyl alkyl
ET₃N: triethylamine
DMAP: 4-dimethylamoniopyridine
DCM: dichloromethane
DMF: dimethylformamide
ACN: acetonitrile

Triethylamine and 4-dimethylamoniopyridine are sequentially added to a mixed solution of dichloromethane and diol. Then, Compound A is added at 0°C thereto and the mixture is stirred at room temperature for 2 hours. After completion of the reaction, the reaction mixture is purified to obtain Compound B. Triethylamine is added to the mixed solution of dichloromethane and Compound B, and acryloyl chloride is added thereto at 0°C. The mixture is stirred at room temperature for 2 hours, and after completion of the reaction, the reaction mixture is purified to obtain Compound C. Potassium carbonate (K₂CO₃) and Compound E are added to the mixed solution of dimethylformamide and Compound D at room temperature. The mixture is stirred at 70°C for 16 hours, and after completion of the reaction, the reaction mixture is purified to obtain Compound F. Hydrazine hydrate is added to a mixed solution of methanol and Compound F at room temperature, and the mixture is stirred at room temperature for 48 hours. After completion of the reaction, the reaction mixture is purified to obtain Compound G. Hydroquinone is added to a mixed solution of Compound G, acetonitrile, and Compound C at room temperature. The reaction mixture is stirred at 70°C for 48 hours, and after completion of the reaction, the reaction mixture is purified to obtain a compound of Formula (I).

The progress of individual reactions according to Reaction Scheme 1 may be monitored by TLC. 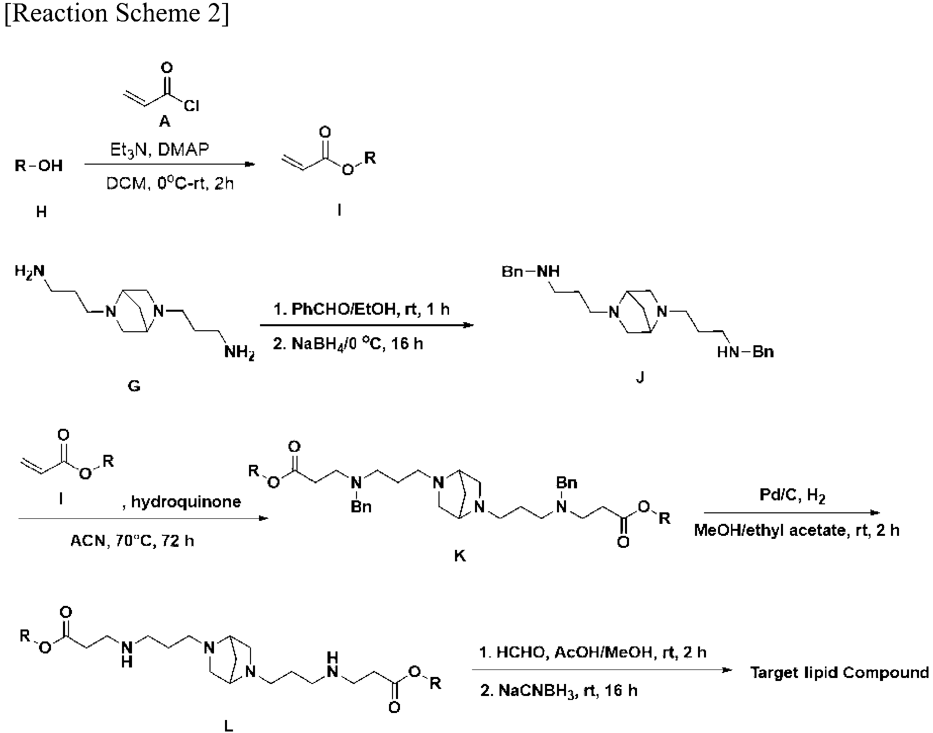 R: linear alkyl, branched alkyl, or oxycarbonyl alkyl

Benzaldehyde and molecular sieves are added to a mixed solution of ethanol and Compound G, and the mixture is stirred at room temperature for 1 hour. Sodium borohydride (NaBH₄) is added to the reaction mixture at 0°C, and the mixture is stirred for 16 hours. The reaction mixture is purified to obtain Compound J.

Separately, triethylamine and 4-dimethylammoniopyridine are added to a mixed solution of dichloromethane and Compound H, and Compound A is added at 0°C. The mixture is stirred at room temperature for 2 hours, and after completion of the reaction, the reaction mixture is purified to obtain Compound I.

Compound J and hydroquinone in acetonitrile are added to the Compound I solution at room temperature. The reaction mixture is stirred at 70°C for 72 hours. After completion of the reaction, the reaction mixture is purified to obtain Compound K. Ethyl acetate and palladium/carbon (Pd/C) are added to a mixed solution of methanol and Compound K, and the mixture is stirred at room temperature for 2 hours under a hydrogen gas balloon pressure (60 psi). After completion of the reaction, the reaction mixture is purified to obtain Compound L. Paraformaldehyde and acetic acid are added to a mixed solution of methanol and Compound L and the mixture was stirred at room temperature for 2 hours. Sodium cyanoborohydride (NaCNBH₃) is added thereto and the mixture is stirred at room temperature for 16 hours. After completion of the reaction, the reaction mixture is purified to obtain a compound of Formula (I). The progress of individual reactions according to Reaction Scheme 2 may be monitored by TLC. 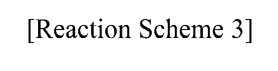 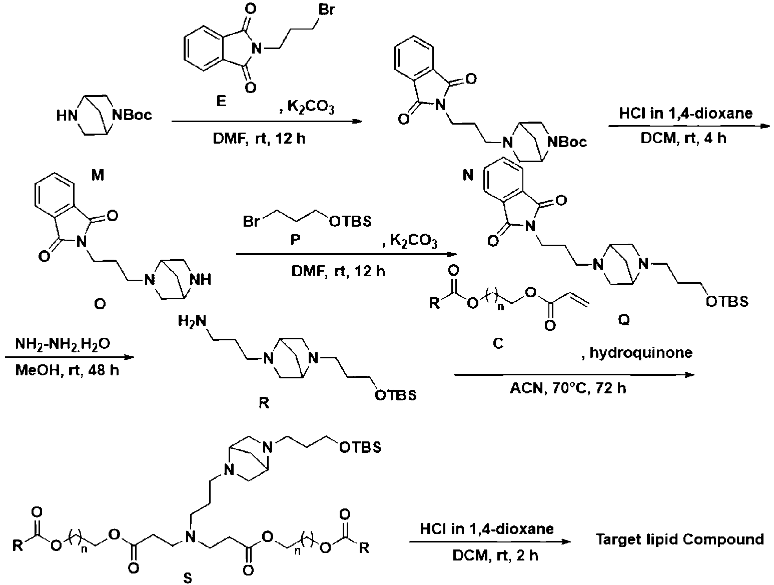 R: linear alkyl, branched alkyl, or oxycarbonyl alkyl

Potassium carbonate and Compound E are added to a mixed solution of dimethylformamide and Compound M at room temperature, and the mixture is stirred at room temperature for 12 hours. After completion of the reaction, the reaction mixture is purified to obtain Compound N. 1,4-Dioxane-HCl (4 M solution) is added to a mixed solution of dichloromethane and Compound N at room temperature, and the mixture is stirred for 4 hours. After completion of the reaction, the reaction mixture is concentrated to obtain Compound O. Potassium carbonate and Compound P are added to a mixed solution of dimethylformamide and Compound O at room temperature, and the mixture is stirred at room temperature for 12 hours. After completion of the reaction, the reaction mixture is purified to obtain Compound Q. Hydrazine hydrate is added to a mixed solution of methanol and Compound Q, and the mixture is stirred at room temperature for 48 hours. After completion of the reaction, the reaction mixture is purified to obtain Compound R. Compound C and hydroquinone in acetonitrile are added to a solution of Compound R at room temperature. The mixture is stirred at 70°C for 72 hours. After completion of the reaction, the reaction mixture is purified to obtain Compound S. 1,4-Dioxane hydrochloric acid is added to a mixed solution of dichloromethane and Compound S at 0°C, and the mixture is stirred at room temperature for 2 hours. After completion of the reaction, the reaction mixture is purified to obtain a compound of Formula (I) or (IV). The progress of individual reactions according to Reaction Scheme 3 may be monitored by TLC.

In Reaction Schemes 1 to 3 above, for the purification of Compounds B to S formed in the process of preparing the compound of Formula (I) or (IV), one or more methods used in the purification of compounds in the field of technology of the subject application, for example, filtration (*e.g*., filtration of celite), extraction (*e.g*., extraction using ethyl acetate or dichloromethane), chromatography (*e.g*., preparative high performance liquid chromatography (prep-HPLC), NH-silica gel (100 to 200 mesh) column chromatography), *etc*. may be used in combination. Additionally, the compound of Formula (I) or (IV) prepared in Reaction Schemes 1 to 3 above may be separated and purified using one or more of the methods used in the field of the subject application for separating and purifying compounds, for example, recrystallization, chromatography, *etc*.

### Lipid nanoparticles

In one aspect of the present invention, the present invention may be lipid nanoparticles including the ionizable lipid compound or a salt thereof, a helper lipid, cholesterol, and a PEG lipid. The helper lipid may be a phospholipid. The phospholipid, which is a lipid including a phosphate group and one or more fatty acid chains, may include one or more multiple bonds, and may interact with one or more negatively charged phospholipids in a membrane (*e.g*., a cell membrane). As the phospholipid, any phospholipid that can promote the fusion of lipid nanoparticles may be used without limitation. As the phospholipid, one or more phospholipids selected from the group consisting of DOPE (dioleoylphosphatidylethanolamine; 1,2-dioleoylsn-glycero-3-phosphoethanolamine), distearoylphosphatidylcholine (DSPC), POPC (palmitoyloleoylphosphatidylcholine; 1-palmitoyl-2-oleoyl-sn-glycero-3-phosphocholine), egg phosphatidylcholine (EPC), dioleoylphosphatidylcholine (DOPC), dipalmitoylphosphatidylcholine (DPPC), dioleoylphosphatidylglycerol (DOPG), dipalmitoylphosphatidylglycerol (DPPG), distearoylphosphatidylethanolamine (DSPE), phosphatidylethanolamine (PE), dipalmitoylphosphatidylethanolamine (DPPE), 1-palmitoyl-2-oleoyl-sn-glycero-3-phosphoethanolamine (POPE), 1,2-dioleoyl-sn-glycero-3-[phospho-L-serine] (DOPS), 1,2-dioctadecanoyl-sn-glycero-3-phosphoserine (DSPS; 18:0 PS), 1,2-di-(9Z-octadecenoyl)-sn-glycero-3-phospho-L-serine (DOPS; 18:1 PS), sphingomyelin, *etc*. may be used, and preferably any one or more selected from the group consisting of DSPC, DOPC, DSPE, DOPE, and sphingomyelin may be used, but is not limited thereto. The PEG lipids may include lipids directly conjugated to PEG or linked to PEG via a linker moiety. The linker moiety may be one or more selected from the group consisting of carbonate (-OC(O)O-), succinoyl, phosphate ester (-O-(O)POH-O-), sulfonate ester, amido (-C(O)NH-), amino (-NR-), carbonyl (-C(O)-), carbamate (-NHC(O)O-), urea (-NHC(O)NH-), disulfide (-SS-), ether (-O-), succinyl (-(O)CCH₂CH₂C(O)-), and succinamidyl (-NHC(O)CH₂CH₂C(O)NH-), but is not limited thereto. The lipid linked to the PEG may be ceramide, dimyristoylglycerol (DMG), succinoyl-diacylglycerol (s-DAG), distearoylphosphatidylcholine (DSPC), distearoylphosphatidylethanolamine (DSPE), or cholesterol. The PEG-lipid may include a linker moiety suitable for conjugating PEG to lipid nanoparticles.

Additionally, as the lipid that constitutes the PEG-lipid, the helper lipid of the lipid nanoparticles and/or cholesterol may be used, but any lipid that can be linked to PEG may be used without limitation.

In the lipid nanoparticles of the present invention, the molar ratio, weight ratio, or volume ratio between a combination of the helper lipid, the cholesterol, and the PEG lipid components and the ionizable lipid compound or a salt thereof may be 1:10 to 10:1, but are not limited thereto. Additionally, the molar ratio, weight ratio, or volume ratio between the ionizable lipid compound or a salt thereof and a combination of the helper lipid, the cholesterol, and the PEG lipid components may be 1:10 to 10:1, or 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, or 1:10.

The molar ratio, weight ratio, or volume ratio between a combination of the helper lipid, the cholesterol, and the PEG lipid components and the ionizable lipid compound or a salt thereof, which constitute the lipid nanoparticles of the present invention, may be 1:1 to 4:1, and preferably 1:1 or 4:1.

The lipid nanoparticles of the present invention may include 5 to 20 mol% of a helper lipid, 20-60 mol% of cholesterol, and 0.5-5 mol% of PEG-lipid, or the lipid nanoparticles of the present invention may include 20 to 80 mol% of the above-mentioned ionizable lipid compound (*e.g*., a compound of Formula (I) or (IV) above) or a salt thereof, 0 to 20 mol% of the helper lipid, 20 to 65 mol% of the cholesterol, and 0 to 5 mol% of the PEG lipid, in which the helper lipid may be a phospholipid. In particular, mol% means the percentage of the number of moles of a specific component divided by the sum of the number of moles of the entire components.

### Composition

In one aspect of the present invention, the present invention may be a composition including an ionic drug and (i) the ionizable lipid compound or a salt thereof, or a composition including an ionic drug and (ii) the lipid nanoparticles.

The ionic drug may be one or more selected from the group consisting of nucleic acids, nucleic acid-based drugs, peptides, protein drugs, protein-nucleic acid constructs, and ionic biopolymer-drug conjugates.

The nucleic acid may be one or more selected from the group consisting of an antisense oligonucleotide (ASO), short interfering RNA (siRNA), micro RNA (miRNA), self-amplifying RNA (SAM), circular RNA, messenger RNA (mRNA), crRNA, tracrRNA, single guide RNA (sgRNA), transfer RNA (tRNA), asymmetric interference RNA (aiRNA), antagomir, ribozyme, dicer substrate RNA, short hairpin RNA (shRNA), plasmid DNA (pDNA), double stranded DNA (dsDNA), partial double stranded DNA, triple stranded DNA, partial triple stranded DNA, single stranded DNA (ssDNA), single stranded RNA (ssRNA), double stranded RNA (dsRNA), locked nucleic acid (LNA), a peptide nucleic acid (PNA), a miRNA analogue, and anti-miRNA, but is not limited thereto.

The ionic drug may be a therapeutic agent for treating a disease or a prophylactic agent for preventing a disease.

The composition may be used as a pharmaceutical composition for treating diseases.

When compared with compositions including ionic drugs together with conventionally-known ionic lipids (*e.g*., MC3), the composition including an ionic drug and (i) the ionizable lipid compound or a salt thereof, or a composition including an ionic drug and (ii) the lipid nanoparticles may exhibit a biologically equivalent or increased drug delivery effect.

Additionally, when compared with compositions including ionic drugs together with conventionally-known lipids (*e.g*., MC3), the composition including an ionic drug and (i) the ionizable lipid compound or a salt thereof, or a composition including an ionic drug and (ii) the lipid nanoparticles may have a biologically equivalent or increased effect of disease treatment or prevention or improved immunogenicity.

The composition may include conventional non-toxic pharmaceutically acceptable additives. The additives that can be used in the composition of the present invention may include, for example, additives include sweeteners, binders, solubilizers, dissolution aids, humectants, emulsifiers, isotonic agents, adsorbents, disintegrants, antioxidants, preservatives, lubricants, fillers, fragrances, *etc*., and may include, for example, lactose, dextrose, sucrose, mannitol, sorbitol, cellulose, glycine, silica, talc, stearic acid, sterin, magnesium stearate, magnesium aluminum silicate, starch, gelatin, gum tragacanth, alginic acid, sodium alginate, methylcellulose, sodium carboxymethylcellulose, agar, water, ethanol, polyethylene glycol, polyvinylpyrrolidone, sodium chloride, calcium chloride, orange essence, strawberry essence, vanilla flavor, *etc*.

The composition of the present invention may be prepared in various oral dosage forms (*e.g*., tablets, pills, powders, capsules, syrups, and emulsions, *etc*.) or parenteral dosage forms (*e.g*., intramuscular, intravenous, and subcutaneous administration).

When the composition of the present invention is formulated into an oral administration form, as additives, cellulose, calcium silicate, corn starch, lactose, sucrose, dextrose, calcium phosphate, stearic acid, magnesium stearate, calcium stearate, gelatin, talc, surfactants, suspending agents, emulsifiers, diluents, *etc*. may be used.

Additionally, when the composition of the present invention is formulated into an injection form, as additives, water, saline, an aqueous glucose solution, an aqueous sugar-like solution, alcohol, glycol, ether, oil, fatty acid, fatty acid ester, glyceride, surfactants, suspending agents, emulsifiers, *etc.* may be used.

### Mode for Carrying Out the Invention

Hereinafter, in order to help understand the present invention, the present invention will be described in detail through embodiments and the like. However, the embodiments according to the present invention may be modified in various different forms, and the scope of the present invention should not be construed as being limited to the following embodiments.

### [Preparation Example] Preparation of ionizable lipids]

The following Preparation Examples are merely illustrative of how the novel ionizable lipid compounds of the present invention can be prepared and are not intended to limit the scope of the present invention as defined in the appended claims.

### Preparation Example 1. Preparation of ionizable lipid compounds 1 to 13

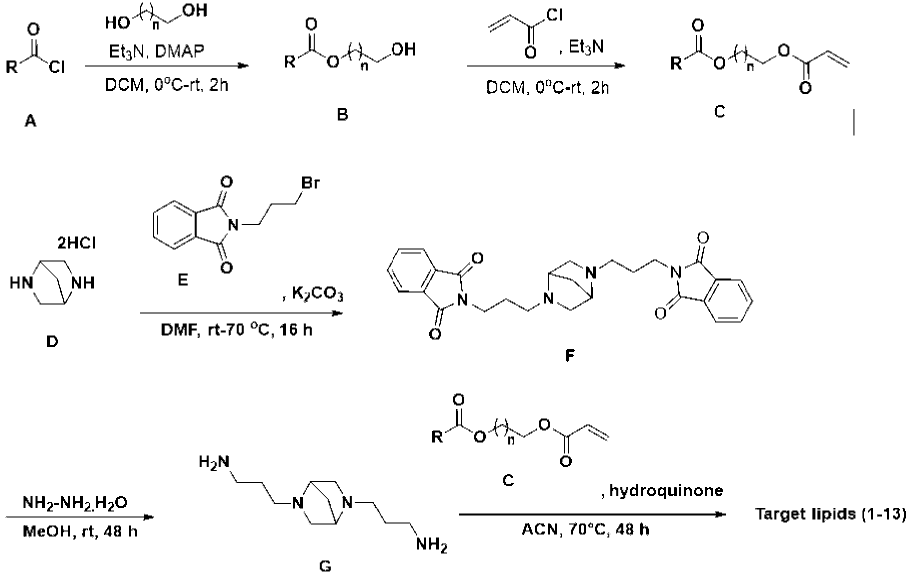

R: linear alkyl, branched alkyl, or oxycarbonyl alkyl

### [Step 1] Synthesis of Compound B

Trimethylamine (1.0 eq.) and 4-dimethylamoniopyridine (0.1 eq.) were sequentially added to a stirred solution of dichloromethane and diol (1.2 eq.), Compound A (1.0 eq.) was added dropwise thereto at 0°C, and the mixture was stirred at room temperature for 2 hours. The reaction progress was monitored by the TLC method. After completion of the reaction, the reaction mixture was diluted with water and extracted twice with dichloromethane. The combined organic layer was dried over anhydrous sodium sulfate (Na₂SO₄), filtered, and the filtrate was concentrated under reduced pressure to obtain a crude compound. The crude compound was purified by silica gel (100-200 mesh) column chromatography to obtain Compound B.

### [Step 2] Synthesis of Compound C

Triethylamine (2.0 eq.) was added to a stirred solution of dichloromethane and Compound B (1.0 eq.), and acryloyl chloride (1.2 eq.) was added dropwise thereto at 0°C. The mixture was stirred at room temperature for 2 hours. The reaction progress was monitored by the TLC method. After completion of the reaction, the reaction mixture was diluted with water and extracted twice with dichloromethane. The combined organic layers were dried over anhydrous sodium sulfate, filtered, and the filtrate was concentrated under reduced pressure to obtain a crude compound. The crude compound was purified by silica gel (100-200 mesh) column chromatography to obtain Compound C.

### [Step 3] Synthesis of Compound F

Potassium carbonate (K₂CO₃) (6 eq.) and Compound E (2.5 eq.) were added to a stirred solution of dimethylformamide and Compound D (1.0 eq.) at room temperature. The mixture was stirred at 70°C for 16 hours. The reaction progress was monitored by the TLC method. After completion of the reaction, the reaction mixture was quenched with water and extracted twice with ethyl acetate. The combined organic layers were dried over anhydrous sodium sulfate, filtered, and the filtrate was concentrated under reduced pressure to obtain a crude compound. The crude compound was purified by silica gel (100-200 mesh) column chromatography to obtain Compound F.

### [Step 4] Synthesis of Compound G

Hydrazine hydrate (2.0 eq.) was added to a stirred solution of methanol and Compound F (1.0 eq.) at room temperature, and the mixture was stirred at room temperature for 48 hours. The reaction progress was monitored by the TLC method. After completion of the reaction, the reaction mixture was filtered through celite and washed with methanol. The filtrate was concentrated under reduced pressure to obtain Compound G.

### [Step 5] Synthesis of ionizable lipid compounds 1 to 13

A catalytic amount of hydroquinone (0.1 eq.) was added to a stirred mixture of compound G (1.0 eq.), acetonitrile, and Compound C (4.0 eq.) at room temperature. The reaction mixture was heated to 70°C and stirred at 70°C for 48 hours. The reaction progress was monitored by the TLC method. After completion of the reaction, the reaction mixture was diluted with water and extracted with ethyl acetate. The combined organic layers were dried over anhydrous sodium sulfate, filtered, and the filtrate was concentrated under reduced pressure to obtain a crude compound. The crude compounds were purified by silica gel (100-200 mesh) column chromatography to obtain ionizable lipid compounds 1 to 13.

### Preparation Example 2. Preparation of ionizable lipid compound 14

R: linear alkyl, branched alkyl, or oxycarbonyl alkyl

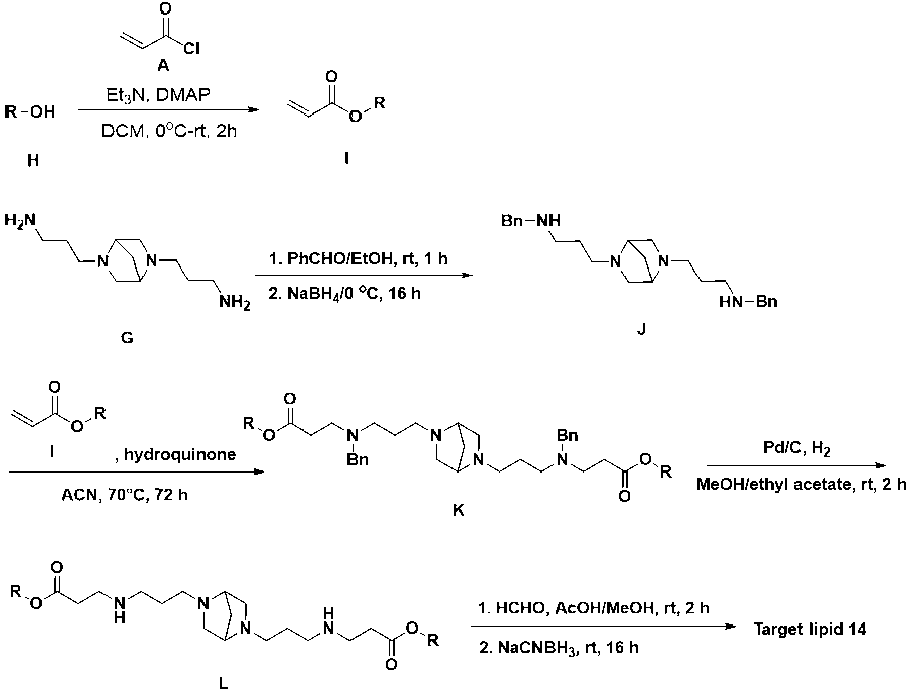

### [Step 1] Synthesis of Compound I

Triethylamine (1.0 eq.) and 4-dimethylamoniopyridine (0.1 eq.) were added to a stirred solution of dichloromethane and Compound H (1.2 eq.), and Compound A (1.0 eq.) was added dropwise thereto at 0°C. The mixture was stirred at room temperature for 2 hours. The reaction progress was monitored by the TLC method. After completion of the reaction, the reaction mixture was diluted with water and extracted twice with dichloromethane. The combined organic layers were dried over anhydrous sodium sulfate, filtered, and the filtrate was concentrated under reduced pressure to obtain a crude compound. The crude compound was purified by silica gel (100-200 mesh) column chromatography to obtain Compound I.

### [Step 2] Synthesis of Compound J

Benzaldehyde (2.0 eq.) and molecular sieves were added to a stirred solution of ethanol and Compound G (1.0 eq.), and the mixture was stirred at room temperature for 1 hour. Then, sodium borohydride (NaBH₄) (6 eq.) was added to the mixture at 0°C, and the mixture was stirred for 16 hours. The reaction progress was monitored by the TLC method. After completion of the reaction, the reaction mixture was filtered, and the filtrate was concentrated under reduced pressure to obtain a crude compound. The residue of the crude compound was quenched with aqueous HCl and washed twice with dichloromethane. The separated aqueous layer was basified to pH 8 using a NaHCO₃ solution and extracted twice with 10% methanol in dichloromethane. The combined organic layers were dried over anhydrous sodium sulfate, filtered, and the filtrate was concentrated under reduced pressure to obtain Compound J.

### [Step 3] Synthesis of Compound K

Compound J (1.0 eq.) and hydroquinone in acetonitrile (0.1 eq.) were added to a stirred solution of Compound I (2.2 eq.) at room temperature. The reaction mixture was heated to 70°C and stirred at 70°C for 72 hours. The reaction progress was monitored by the TLC method. After completion of the reaction, the reaction mixture was concentrated under reduced pressure to obtain a crude compound. The crude compound was purified by NH-silica gel (100-200 mesh) column chromatography to obtain Compound K.

### [Step 4] Synthesis of Compound L

Compound K (1.0 eq.), ethyl acetate, and palladium/carbon (Pd/C) (0.1 eq.) were added to a stirred solution of methanol, and the mixture was stirred at room temperature for 2 hours under a hydrogen gas balloon pressure (60 psi). The reaction progress was monitored by the TLC method. After completion of the reaction, the reaction mixture was filtered through celite and washed with ethyl acetate. The filtrate was concentrated under reduced pressure to obtain Compound L.

### [Step 5] Synthesis of ionizable lipid compound 14

Paraformaldehyde (10.0 eq.) and one drop of acetic acid were added to a stirred solution of methanol and Compound L (1.0 eq.), and the mixture was stirred at room temperature for 2 hours. Sodium cyanoborohydride (NaCNBH₃) (10.0 eq.) was added and the mixture was stirred at room temperature for 16 hours. The reaction progress was monitored by the TLC method. The reaction mixture was diluted with water and extracted twice with dichloromethane and 5% methanol. The combined organic layers were dried over anhydrous sodium sulfate, filtered, and the filtrate was concentrated under reduced pressure to obtain a crude compound. The crude compound was purified by NH-silica gel (100-200 mesh) column chromatography to obtain ionizable lipid compound 14.

### Preparation Example 3. Preparation of ionizable lipid compound 15

R: linear alkyl, branched alkyl, or oxycarbonyl alkyl

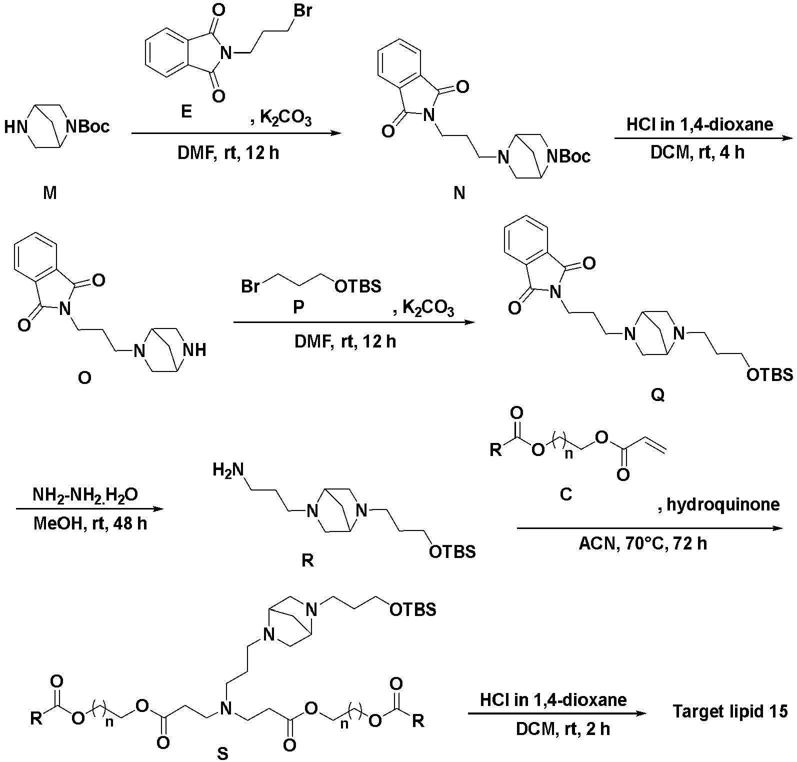

### [Step 1] Synthesis of Compound N

Potassium carbonate (2.50 eq.) and Compound E (1.30 eq.) were added to a stirred solution of dimethylformamide and Compound M (1.0 eq.) at room temperature, and the mixture was stirred at room temperature for 12 hours. The reaction progress was monitored by the TLC method. After completion of the reaction, the reaction mixture was quenched with water and extracted twice with ethyl acetate. The combined organic layers were dried over anhydrous sodium sulfate, filtered, and the filtrate was concentrated under reduced pressure to obtain Compound N.

### [Step 2] Synthesis of Compound O

1,4-Dioxane-HCl (4 M solution) was added to a stirred solution of dichloromethane and Compound N (1.0 eq.) at room temperature, and the mixture was stirred for 4 hours. The reaction progress was monitored by the TLC method. After completion of the reaction, the reaction mixture was concentrated under reduced pressure to obtain Compound O.

### [Step 3] Synthesis of Compound Q

Potassium carbonate (2.50 eq.) and Compound P (1.30 eq.) were added to a stirred solution of dimethylformamide and Compound O (1.0 eq.) at room temperature, and the mixture was stirred at room temperature for 12 hours. The reaction progress was monitored by the TLC method. After completion of the reaction, the reaction mixture was quenched with water and extracted twice with ethyl acetate. The combined organic layers were dried over anhydrous sodium sulfate, filtered, and the filtrate was concentrated under reduced pressure to obtain Compound Q.

### [Step 4] Synthesis of Compound R

Hydrazine hydrate (2.0 eq.) was added to a stirred solution of methanol and Compound Q (1.0 eq.), and the mixture was stirred at room temperature for 48 hours. The reaction progress was monitored by the TLC method. After completion of the reaction, the reaction mixture was filtered through celite and washed with methanol. The filtrate was concentrated under reduced pressure to obtain Compound R.

### [Step 5] Synthesis of Compound S

Compound C (2.50 eq.) and hydroquinone (0.1 eq.) in acetonitrile were added to a stirred solution of Compound R (1.0 eq.) at room temperature. The mixture was heated to 70°C and stirred at 70°C for 72 hours. The reaction progress was monitored by the TLC method. After completion of the reaction, the reaction mixture was diluted with water and extracted with ethyl acetate. The combined organic layers were dried over anhydrous sodium sulfate, filtered, and the filtrate was concentrated under reduced pressure to obtain a crude compound. The crude compound was purified by NH-silica gel (100-200 mesh) column chromatography to obtain Compound S.

### [Step 6] Synthesis of ionizable lipid compound 15

4 M 1,4-Dioxane hydrochloric acid was added to a stirred solution of dichloromethane and Compound S (1.0 eq.) at 0°C, and the mixture was stirred at room temperature for 2 hours. The reaction progress was monitored by the TLC method. After completion of the reaction, the reaction mixture was quenched with a NaHCO₃ solution and extracted twice with dichloromethane. The combined organic layers were dried over anhydrous sodium sulfate, filtered, and the filtrate was concentrated under reduced pressure to obtain a crude compound. The crude compound was purified by preparative liquid chromatography (prep-HPLC) to obtain ionizable lipid compound 15.

The structural formulas of ionizable lipid compounds 1 to 15 synthesized in Preparation Examples 1 to 3 above are listed in Table 1 below.

**[Table 1]**

| | Structure |
|---|---|
| Lipid 1 | ((3,3',3'',3‴-(((2,5-Diazabicyclo[2.2.1]heptane-2,5-diyl)bis(propane-3,1-diyl))bis(azanetriyl)) tetrakis(propanoyl))tetrakis(oxy))tetrakis(butane-4,1-diyl) tetranonanoate |
| | 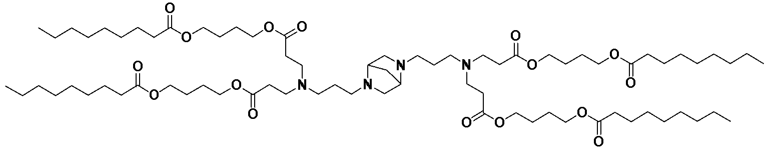 |
| Lipid 2 | Tetrakis(2-ethylhexyl) 3,3',3'',3‴-(((2,5-diazabicyclo[2.2.1]heptane-2,5-diyl)bis(propane-3,1-diyl))bis(azanetriyl))tetrapropionate |
| | 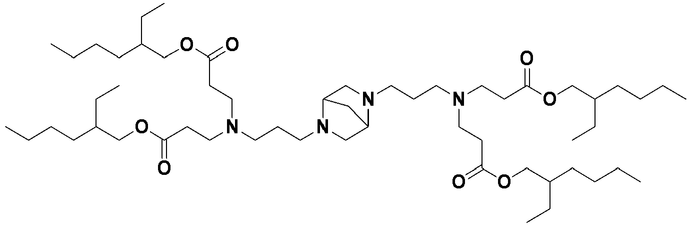 |
| Lipid 3 | Tetrakis(4-(2-cyclohexylacetoxy)butyl) 3,3',3",,3‴'-(((2,5-diazabicyclo[2.2.1]heptane-2,5-diyl)bis (propane-3,1-diyl))bis(azanetriyl))tetrapropionate |
| | 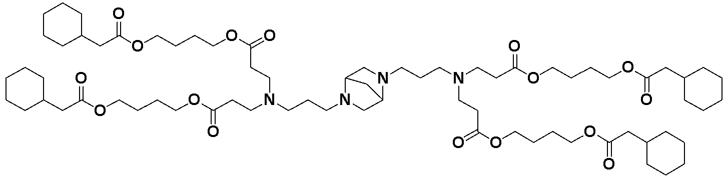 |
| Lipid 4 | ((3,3',3'',3‴(((2,5-Diazabicyclo[2.2.1]heptane-2,5-diyl)bis(propane-3,1-diyl))bis(azanetriyl)) tetrakis(propanoyl))tetrakis(oxy))tetrakis(butane-4,1-diyl) tetraheptanoate |
| | 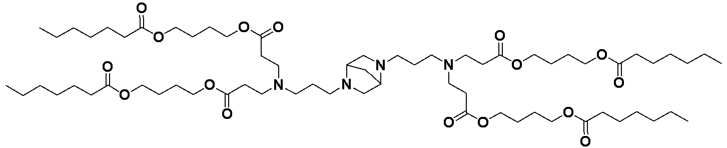 |
| Lipid 5 | ((3,3',3'',3‴-(((2,5-Diazabicyclo[2.2.1]heptane-2,5-diyl)bis(propane-3,1-diyl))bis(azanetriyl)) tetrakis(propanoyl))tetrakis(oxy))tetrakis(ethane-2,1-diyl) tetraheptanoate |
| | 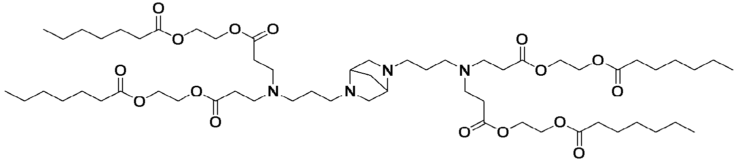 |
| Lipid 6 | ((3,3',3'',3‴-(((2,5-Diazabicyclo[2.2.1]heptane-2,5-diyl)bis(propane-3,1-diyl))bis(azanetriyl)) tetrakis(propanoyl))tetrakis(oxy))tetrakis(butane-4,1-diyl) tetratridecanoate |
| | 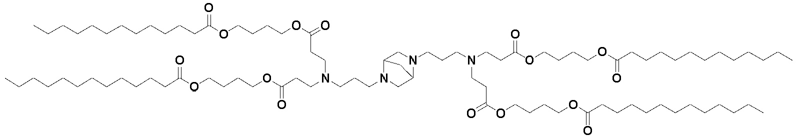 |
| Lipid 7 | Tetrakis(2-ethylhexyl) 4,4',4",4‴-((3,3',3",,3‴-(((2,5-diazabicyclo[2.2.1]heptane-2,5-diyl)bis (propane-3,1-diyl))bis(azanetriyl))tetrakis(propanoyl))tetrakis(oxy))tetrabutyrate |
| | 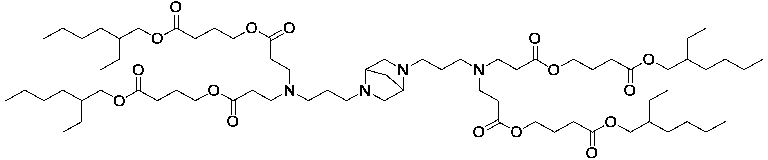 |
| Lipid 8 | Tetrakis(2-ethylhexyl) 6,6',6",6‴-((3,3',3",3‴-(((2,5-diazabicyclo[2.2.1]heptane-2,5-diyl)bis (propane-3,1-diyl))bis(azanetriyl))tetrakis(propanoyl))tetrakis(oxy))tetrahexanoate |
| | 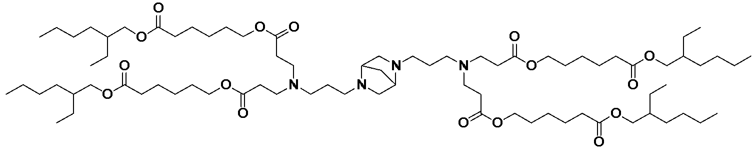 |
| Lipid 9 | Tetrakis(2-propylhexyl) 4,4',4",4‴-((3,3',3",3‴-(((2,5-diazabicyclo[2.2.1]heptane-2,5-diyl)bis (propane-3,1-diyl))bis(azanetriyl))tetrakis(propanoyl))tetrakis(oxy))tetrabutyrate |
| | 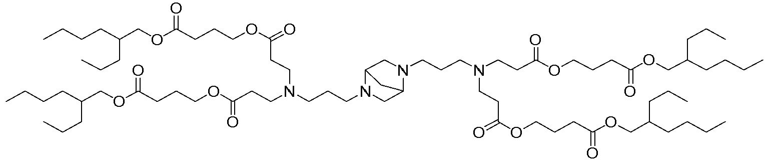 |
| Lipid 10 | Tetrakis(2-ethylpentyl) 4,4',4",4‴-((3,3',3",3‴-(((2,5-diazabicyclo[2.2.1]heptane-2,5-diyl)bis (propane-3,1-diyl))bis(azanetriyl))tetrakis(propanoyl))tetrakis(oxy))tetrabutyrate |
| | 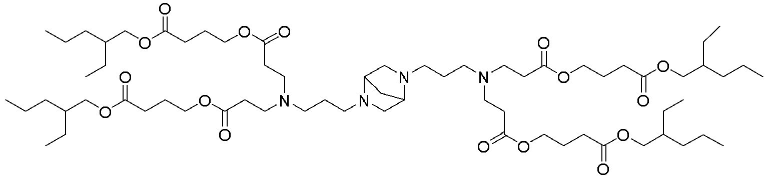 |
| Lipid 11 | Tetrakis(2-ethylpentyl) 6,6',6",6‴-((3,3',3",3‴-(((2,5-diazabicyclo[2.2.1]heptane-2,5-diyl)bis (propane-3,1-diyl))bis(azanetriyl))tetrakis(propanoyl))tetrakis(oxy))tetrahexanoate |
| | 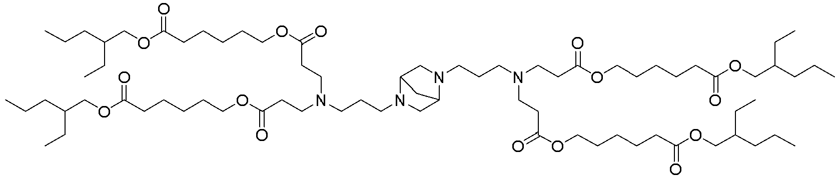 |
| Lipid 12 | Tetra((Z)-non-2-en-1-yl) 9,9',9",9‴-(((2,5-diazabicyclo[2.2.1]heptane-2,5-diyl)bis(propane-3,1-diyl))bis(azanetriyl))tetranonanoate |
| | 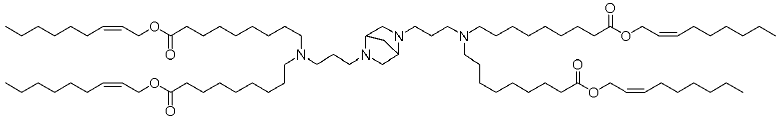 |
| Lipid 13 | (((3,3',3'',3‴-(((2,5-Diazabicyclo[2.2.1]heptane-2,5-diyl)bis(propane-3,1-diyl))bis(azanetriyl))tetrakis(propanoyl))tetrakis(oxy))tetrakis(methylene)) tetrakis(propane-2,1,3-triyl) octahexanoate |
| | 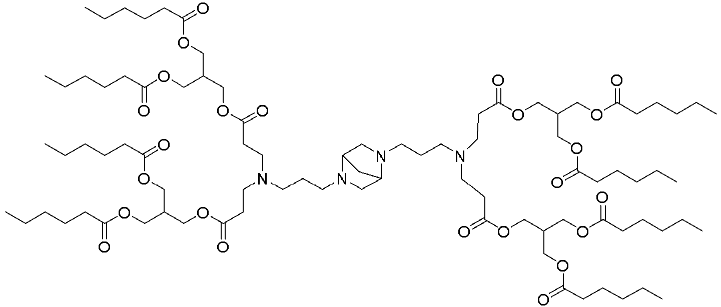 |
| Lipid 14 | Di(henicosan-10-yl) 3,3'-(((2,5-diazabicyclo[2.2.1]heptane-2,5-diyl)bis(propane-3,1-diyl))bis (methylazanediyl))dipropionate |
| | 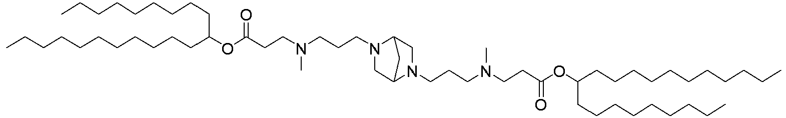 |
| Lipid 15 | ((3,3'-((3-(5-(3-hydroxypropyl)-2,5-diazabicyclo[2.2.1]heptan-2-yl)propyl)azanediyl)bis (propanoyl))bis(oxy))bis(hexane-6,1-diyl) bis(2-hexyldecanoate) |
| | 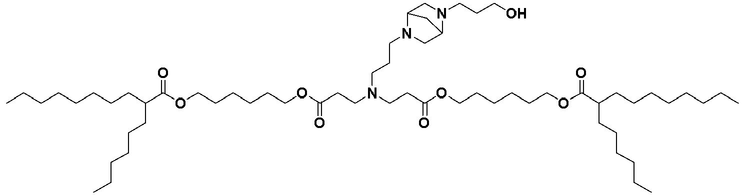 |

Meanwhile, ¹H NMR spectra of the synthesized Lipids 1 to 15 were measured using either a Bruker Ultrashield 400 or a spectrometer. Chemical shifts were expressed in parts per million (ppm, δ units). The coupling constant is in hertz (Hz), and the splitting pattern describes the diversity and is indicated by singlet (s), doublet (d), triplet (t), quartet (q), quintet (quint), multiplet (m), or broad (br). The ¹H NMR (400 MHz, CDCl₃) data of Lipid 1 to Lipid 15 are shown in Table 2.

**[Table 2]**

| Compound | ¹H NMR (400 MHz, CDCl₃) |
|---|---|
| Lipid 1 | ((3,3',3",3‴-(((2,5-Diazabicyclo[2.2.1]heptane-2,5-diyl)bis(propane-3,1-diyl))bis(azanetriyl)) tetrakis(propanoyl))tetrakis(oxy))tetrakis(butane-4,1-diyl) tetranonanoate |
| | ¹H NMR (400 MHz, DMSO-*d*₆) δ 0.80-0.90 (m, 12H), 1.20-1.30 (m, 40H), 1.40-1.70 (m, 28H), 1.90-2.00 (m, 2H), 2.20-2.29 (m, 8H), 2.30-2.45 (m, 10H), 2.60-3.20 (m, 16H), 4.00-4.10 (m, 20H); LCMS (ESI) *m*/*z* 1349.90 [M+H]⁺. |
| Lipid 2 | Tetrakis(2-ethylhexyl) 3,3',3",3‴-(((2,5-diazabicyclo[2.2.1]heptane-2,5-diyl)bis(propane-3,1-diyl))bis(azanetriyl))tetrapropionate |
| | ¹H NMR (400 MHz, CDCl₃) δ 0.80-0.90 (m, 24H), 1.20-1.40 (m, 34H), 1.50-1.60 (m, 6H), 2.00-2.20 (m, 2H), 2.40-3.51 (m, 30H), 3.93-4.01 (m, 8H); LCMS (ESI) *m*/*z* 950.01 [M+H]⁺. |
| Lipid 3 | Tetrakis(4-(2-cyclohexylacetoxy)butyl) 3,3',3",3‴-(((2,5-diazabicyclo[2.2.1]heptane-2,5-diyl)bis (propane-3,1-diyl))bis(azanetriyl))tetrapropionate |
| | ¹H NMR (400 MHz, CDCl₃) δ 0.90-1.05 (m, 8H), 1.10-1.40 (m, 14H), 1.50-1.68 (m, 28H), 1.70-1.85 (m, 16H), 2.10-2.20 (m, 8H), 2.30-3.00 (m, 28H), 3.20-3.30 (m, 2H), 4.00-4.10 (m, 16H); LCMS (ESI) *m*/*z* 1285.93 [M+H]⁺. |
| Lipid 4 | ((3,3',3",3‴-(((2,5-Diazabicyclo[2.2.1]heptane-2,5-diyl)bis(propane-3,1-diyl))bis(azanetriyl)) |
| | tetrakis(propanoyl))tetrakis(oxy))tetrakis(butane-4,1-diyl) tetraheptanoate |
| | ¹H NMR (400 MHz, DMSO-*d*₆) δ 0.80-0.90 (m, 12H), 1.20-1.25 (m, 26H), 1.30-1.40 (m, 4H), 1.45-1.55 (m, 10H), 1.60-1.70 (m, 18H), 2.20-2.30 (m, 8H), 2.32-2.38 (m, 12H), 2.40-2.45 (m, 4H), 2.60-2.70 (m, 8H), 3.10-3.20 (m, 2H), 4.00-4.10 (m, 16H); LCMS (ESI) *m*/*z* 1237.91 [M+H]⁺. |
| Lipid 5 | ((3,3',3",3‴-(((2,5-Diazabicyclo[2.2.1]heptane-2,5-diyl)bis(propane-3,1-diyl))bis(azanetriyl)) |
| | tetrakis(propanoyl))tetrakis(oxy))tetrakis(ethane-2,1-diyl) tetraheptanoate |
| | ¹H NMR (400 MHz, CDCl₃) δ 0.82-0.98 (m, 12H), 1.25-1.38 (m, 24H), 1.50-1.55 (m, 4H), 1.60-1.68 (m, 12H), 2.28-2.38 (m, 8H), 2.40-2.58 (m, 14H), 2.60-2.80 (m, 12H), 3.20-3.30 (m, 2H), 4.20-4.30 (m, 16H); LCMS (ESI) *m*/*z* 1125.83 [M+H]⁺. |
| Lipid 6 | ((3,3',3",3‴-(((2,5-Diazabicyclo[2.2.1]heptane-2,5-diyl)bis(propane-3,1-diyl))bis(azanetriyl)) |
| | tetrakis(propanoyl))tetrakis(oxy))tetrakis(butane-4,1-diyl) tetratridecanoate |
| | ¹H NMR (400 MHz, CDCl₃) δ 0.80-0.90 (m, 12H), 1.20-1.45 (m, 74H), 1.50-1.60 (m, 6H), 1.65-1.75 (m, 22H), 2.27-2.31 (m, 8H), 2.42-2.78 (m, 28H), 3.24-3.25 (m, 2H), 4.05-4.15 (m, 16H); LCMS (ESI) *m*/*z* 1574.37 [M+H]⁺. |
| Lipid 7 | Tetrakis(2-ethylhexyl) 4,4',4",4‴-((3,3',3",3‴-(((2,5-diazabicyclo[2.2.1]heptane-2,5-diyl)bis (propane-3,1-diyl))bis(azanetriyl))tetrakis(propanoyl))tetrakis(oxy))tetrabutyrate |
| | ¹H NMR (400 MHz, CDCl₃) δ 0.87-0.90 (m, 24H), 1.26-1.39 (m, 34H), 1.53-1.66 (m, 8H), 1.93-1.99 (m, 8H), 2.38-2.78 (m, 36H), 3.20-3.30 (m, 2H), 3.96-4.04 (m, 8H), 4.10 (t, *J* = 6.4 Hz, 8H); LCMS (ESI) *m*/*z* 1293.94 [M+H]⁺. |
| Lipid 8 | Tetrakis(2-ethylhexyl) 6,6',6",6‴-((3,3',3",3‴-(((2,5-diazabicyclo[2.2.1]heptane-2,5-diyl)bis (propane-3,1-diyl))bis(azanetriyl))tetrakis(propanoyl))tetrakis(oxy))tetrahexanoate |
| | ¹H NMR (400 MHz, CDCl₃) δ 0.80-0.90 (m, 24H), 1.20-1.40 (m, 40H), 1.50-1.60 (m, 10H), 1.62-1.70 (m, 18H), 2.28-2.38 (m, 8H), 2.40-2.60 (m, 15H), 2.62-2.68 (m, 4H), 2.70-2.80 (m, 7H), 3.20-3.30 (m, 2H), 3.97-3.99 (m, 8H), 4.04-4.07 (m, 8H); LCMS (ESI) *m*/*z* 1406.29 [M+H]⁺. |
| Lipid 9 | Tetrakis(2-propylhexyl) 4,4',4",4‴-((3,3',3",3‴-(((2,5-diazabicyclo[2.2.1]heptane-2,5-diyl)bis (propane-3,1-diyl))bis(azanetriyl))tetrakis(propanoyl))tetrakis(oxy))tetrabutyrate ¹H NMR (400 MHz, CDCl₃) δ 0.88-0.91 (m, 24H), 1.20-1.50 (m, 42H), 1.48-1.50 (m, 2H), 1.55-1.60 (m, 4H), 1.62-1.65 (m, 6H), 1.94-1.99 (m, 8H), 2.38-2.50 (m, 22H), 2.70-2.80 (m, 10H), 3.20-3.30 (m, 2H), 3.99 (d, J = 6 Hz, 8H), 4.10 (t, J = 6.4 Hz, 8H); LCMS (ESI) *m*/*z* 1350.26 [M+H]⁺. |
| Lipid 10 | Tetrakis(2-ethylpentyl) 4,4',4'',4'''-((3,3',3",3‴-(((2,5-diazabicyclo[2.2.1]heptane-2,5-diyl)bis (propane-3,1-diyl))bis(azanetriyl))tetrakis(propanoyl))tetrakis(oxy))tetrabutyrate |
| | ¹H NMR (400 MHz, CDCl₃) δ 0.80-0.90 (m, 24H), 1.20-1.40 (m, 28H), 1.50-1.55 (m, 4H), 1.60-1.70 (m, 6H), 1.90-2.00 (m, 8H), 2.38-2.48 (m, 22H), 2.64-2.78 (m, 10H), 3.20-3.30 (m, 2H), 3.99-4.12 (m, 16H); LCMS (ESI) *m*/*z* 1237.79 [M+H]⁺. |
| Lipid 11 | Tetrakis(2-ethylpentyl) 6,6',6",6‴-((3,3',3",3‴-(((2,5-diazabicyclo[2.2.1]heptane-2,5-diyl)bis (propane-3,1-diyl))bis(azanetriyl))tetrakis(propanoyl))tetrakis(oxy))tetrahexanoate |
| | ¹H NMR (400 MHz, CDCl₃) δ 0.85-0.95 (m, 24H), 1.25-1.45 (m, 34H), 1.60-1.72 (m, 28H), 2.30-2.35 (m, 8H), 2.40-2.60 (m, 15H), 2.62-2.70 (m, 3H), 2.72-2.80 (m, 6H), 3.20-3.30 (m, 2H), 3.98-4.07 (m, 16H); LCMS (ESI) *m*/*z* 1350.05 [M+H]⁺. |
| Lipid 12 | Tetra((Z)-non-2-en-1-yl) 9,9',9",9‴-(((2,5-diazabicyclo[2.2.1]heptane-2,5-diyl)bis(propane-3,1-diyl))bis(azanetriyl))tetranonanoate |
| | ¹H NMR (400 MHz, CDCl₃) δ 0.80-0.90 (m, 12H), 1.20-1.50 (m, 76H), 1.52-1.60 (m, 12H), 2.10-2.18 (m, 8H), 2.30-2.34 (m, 8H), 2.35-2.60 (m, 14H), 2.65-2.85 (m, 4H), 3.20-3.40 (m, 2H), 4.60-4.70 (m, 8H), 5.49-5.59 (m, 4H), 5.60-5.70 (m, 4H); LCMS (ESI) *m*/*z* 1334.29 [M+H]⁺. |
| Lipid 13 | (((3,3',3",3‴-(((2,5-Diazabicyclo[2.2.1]heptane-2,5-diyl)bis(propane-3,1-diyl))bis(azanetriyl))tetrakis(propanoyl))tetrakis(oxy))tetrakis(methylene ))tetrakis(propane-2,1,3-triyl) octahexanoate |
| | ¹H NMR (400 MHz, MeOD) δ 0.88-0.95 (m, 24H), 1.25-1.40 (m, 32H), 1.55-1.65 (m, 20H), 1.70-1.80 (m, 2H), 2.30-2.40 (m, 16H), 2.42-2.60 (m, 18H), 2.62-2.90 (m, 14H), 3.40-3.45 (m, 2H), 4.16 (d, J = 6 Hz, 24H); LCMS (ESI) *m*/*z* 1637.92 [M+H]⁺. |
| Lipid 14 | Di(henicosan-10-yl) 3,3'-(((2,5-diazabicyclo[2.2.1]heptane-2,5-diyl)bis(propane-3,1-diyl))bis (methylazanediyl))dipropionate |
| | ¹H NMR (400 MHz, CDCl₃) δ 0.80-0.90 (m, 12H), 1.20-1.30 (m, 62H), 1.45-1.52 (m, 8H), 1.55-1.62 (m, 6H), 1.65-1.72 (m, 2H), 2.22 (s, 6H), 2.35-2.50 (m, 10H), 2.50-2.60 (m, 2H), 2.62-2.72 (m, 8H), 3.26 (s, 2H), 4.83 - 4.89 (m, 2H); LCMS (ESI) *m*/*z* 973.99 [M+H]⁺. |
| Lipid 15 | ((3,3'-((3-(5-(3-hydroxypropyl)-2,5-diazabicyclo[2.2.1]heptan-2-yl)propyl)azanediyl)bis (propanoyl))bis(oxy))bis(hexane-6,1-diyl) bis(2-hexyldecanoate) |
| | ¹H NMR (400 MHz, MeOD) δ 0.80-0.90 (m, 16H), 1.20-1.40 (m, 35H), 1.42-1.50 (m, 13H), 1.52-1.75 (m, 17H), 1.76-1.88 (m, 2H), 2.30-2.40 (m, 2H), 2.42_2.50 (m, 6H), 2.52-2.90 (m, 12H), 3.40-3.50 (m, 2H), 3.60-3.70 (m, 2H), 4.06-4.11 (m, 8H); LCMS (ESI) *m*/*z* 1034.96 [M+H]⁺. |

### [Experimental Examples]

### Experimental Example 1. Characteristics of ionizable lipids

### [Preparation of lipid nanoparticles (LNPs)]

For the preparation of LNP, cholesterol purchased from Sigma Aldrich (USA) was used, and 1,2-dimyristoyl-rac-glycero-3-methoxypolyethylene glycol-2000 (DMG-PEG2000) purchased from Avanti Polar Lipids (USA) was used as a PEG lipid. Additionally, 1,2-dioleoyl-sn-glycero-3-phosphoethanolamine (DOPE) and 1,2-distearoyl-sn-glycero-3-PC (DSPC) purchased from Avanti Polar Lipids (USA) were used as helper lipids.

Lipids 1 to 5 and Lipids 8 to 15 synthesized in Preparation Examples above were each dissolved in ethanol together with the DOPE helper lipid, the cholesterol, and the PEG-lipid, and Lipids 1 to 4 and Lipids 8 to 15 synthesized in Preparation Examples above were each dissolved in ethanol together with the DSPC helper lipid, the cholesterol, and the PEG-lipid. When dissolved in ethanol, the mixing ratio of each substance is as described in Table 3.

**[Table 3]**

| LNP | Mixing ratio (mol%) (Ionizable lipid: Helper lipid: Cholesterol: PEG-lipid) |
|---|---|
| F1-type LNP | 50: 10: 38.5: 1.5 |
| F2-type LNP | 26.5: 20: 51.5: 2 |

For preparation of LNPs, NanoAssemblr Ignite^{™} (Precision Nanosystems, Inc., Canada) was used, and a total flow rate (TFR) of 12 mL/min was used. Ethanol from the prepared LNPs was removed using Amicon Ultra Centrifugal Filter, MWCO 10 kDa (Millipore, USA), the buffer was exchanged, and then the LNPs were concentrated. During dilution, concentration, and exchange, the prepared LNPs were diluted with 1X DPBS to remove ethanol, and the LNPs were concentrated at the same time.

### [Method for analyzing physicochemical properties of LNPs]

Particle size, z-average diameter, and polydispersity index (PDI) were analyzed using the Zetasizer Pro (Malvern Instruments, United Kingdom). The "PDI" refers to a ratio describing the homogeneity of the particle size distribution of a system, in which as the PDI approaches closer to 0, it indicates monodisperse, whereas as it approaches closer to 1, it indicates polydisperse.

### [Method for mRNA quantification and measurement of encapsulation efficiency (EE%)]

mRNA Content and EE% were measured using the Ribogreen RNA assay kit (Invitrogen, USA). In brief, the mRNA concentration was measured for the Triton X-100 solution-treated group and untreated group for the prepared mRNA/LNPs, and the ratio of mRNA encapsulated in LNPs among the total mRNA was calculated as EE%.

The z-average diameter, PDI, and EE (%) measurements of the prepared LNPs are listed in Table 4 below, and the z-average diameter and EE (%) are plotted in Figs. 1 and 2, respectively.

**[Table 4]**

| **LNP** | **Z-average (nm)** | **PDI** | **EE (%)** |
|---|---|---|---|
| LNP including ionizable lipid compound 1 (Lipid 1-(F1)) | 94.68 | 0.0263 | 97.96 |
| LNP including ionizable lipid compound 1 (Lipid 1-(F2)) | 106.3 | 0.07377 | 98.81 |
| LNP including ionizable lipid compound 2 (Lipid 2-(F1)) | 100.7 | 0.0099 | 98.98 |
| LNP including ionizable lipid compound 2 (Lipid 2-(F2)) | 87.42 | 0.04302 | 98.91 |
| LNP including ionizable lipid compound 3 (Lipid 3-(F1)) | 139 | 0.0053 | 95.99 |
| LNP including ionizable lipid compound 3 (Lipid 3-(F2)) | 100.9 | 0.0419 | 96.64 |
| LNP including ionizable lipid compound 4 (Lipid 4-(F1)) | 88.83 | 0.1135 | 93.29 |
| LNP including ionizable lipid compound 4 (Lipid 4-(F2)) | 98.52 | 0.03759 | 95.97 |
| LNP including ionizable lipid compound 5 (Lipid 5-(F1)) | 87.45 | 0.1265 | 94.11 |
| LNP including ionizable lipid compound 8 (Lipid 8-(F1)) | 124.7 | 0.0145 | 98.53 |
| LNP including ionizable lipid compound 8 (Lipid 8-(F2)) | 96 | 0.0558 | 98.84 |
| LNP including ionizable lipid compound 9 (Lipid 9-(F1)) | 119.1 | 0.0211 | 98.61 |
| LNP including ionizable lipid compound 9 (Lipid 9-(F2)) | 105.3 | 0.0648 | 98.71 |
| LNP including ionizable lipid compound 10 (Lipid 10-(F1)) | 138.1 | 0.0264 | 98.27 |
| LNP including ionizable lipid compound 10 (Lipid 10-(F2)) | 100.4 | 0.0727 | 98.83 |
| LNP including ionizable lipid compound 11 (Lipid 11-(F1)) | 127.3 | 0.0110 | 98.45 |
| LNP including ionizable lipid compound 11 (Lipid 11-(F2)) | 98.37 | 0.0486 | 98.79 |
| LNP including ionizable lipid compound 12 (Lipid 12-(F1)) | 117.3 | 0.04611 | 100.00 |
| LNP including ionizable lipid compound 12 (Lipid 12-(F2)) | 92.26 | 0.0475 | 100.00 |
| LNP including ionizable lipid compound 13 (Lipid 13-(F1)) | 114.2 | 0.02551 | 98.76 |
| LNP including ionizable lipid compound 13 (Lipid 13-(F2)) | 88.59 | 0.0486 | 99.21 |
| LNP including ionizable lipid compound 14 (Lipid 14-(F1)) | 103.5 | 0.01557 | 99.96 |
| LNP including ionizable lipid compound 14 (Lipid 14-(F2)) | 109 | 0.0762 | 100.00 |
| LNP including ionizable lipid compound 15 (Lipid 15-(F1)) | 129.1 | 0.01417 | 100.00 |
| LNP including ionizable lipid compound 15 (Lipid 15-(F2)) | 112.7 | 0.1468 | 100.00 |

As can be seen from Table 4 above, the LNPs including the present ionizable lipid compounds were confirmed to have a z-average diameter of 130 nm or less and a narrow particle size distribution with a PDI of less than 0.12.

### Experimental Example 2. Nucleic acid delivery efficacy of novel ionizable lipid compounds (in vitro)

In order to confirm the nucleic acid delivery effect of the new ionizable lipid compound, a transfection efficiency test was performed using HEK293T cells.

The human embryonic kidney-293T (HEK293T, ATCC) cell line was seeded at 15,000 cells/well in a 96-well microplate using DMEM media (10% fetal bovine serum (FBS), Gibco), including1% penicillin-streptomycin, and incubated overnight (37°C, 5% CO₂ incubator).

As a test LNP to confirm the nucleic acid delivery efficacy, the LNPs including Lipid 1-(F1), Lipid 1-(F2), Lipid 9-(F1), Lipid 9-(F2), Lipid 11-(F1), Lipid 11-(F2), Lipid 12-(F1), Lipid 12-(F2), or Lipid 15-(F1) in Table 3 were used. As a negative control, firefly luciferase mRNA (free mRNA) without LNP was used, and as a positive control, an LNP including the ionizable amino lipid MC3 (also called as DLin-MC3-DMA) (hereinafter, MC3 LNP) was used.

10 µL LNP for tests and samples corresponding to 25 ng of free mRNA were treated per well and incubated for 24 hours. After 24 hours of transfection, Bright-Glo^{™} Luciferase Assay System (Promega, USA) solution was treated followed by the measurement of luciferase activity.

The average RLU measured for each LNP is described in Table 5 below, and each value is plotted in Fig. 3.

**[Table 5]**

| | **Average RLU** |
|---|---|
| LNP including ionizable lipid compound 1 (Lipid 1-(F1)) | 2.61 × 10⁷ |
| LNP including ionizable lipid compound 1 (Lipid 1-(F2)) | 1.85 × 10⁷ |
| LNP including ionizable lipid compound 9 (Lipid 9-(F1)) | 2.43 × 10⁷ |
| LNP including ionizable lipid compound 9 (Lipid 9-(F2)) | 1.74 × 10⁷ |
| LNP including ionizable lipid compound 11 (Lipid 11-(F1)) | 2.81 × 10⁷ |
| LNP including ionizable lipid compound 11 (Lipid 11-(F2)) | 1.54 × 10⁷ |
| LNP including ionizable lipid compound 12 (Lipid 12-(F1)) | 1.12 × 10⁸ |
| LNP including ionizable lipid compound 12 (Lipid 12-(F2)) | 4.66 × 10⁷ |
| LNP including ionizable lipid compound 15 (Lipid 15-(F1)) | 2.23 × 10⁷ |
| MC3 LNP | 1.46 × 10⁶ |
| Control Group (free mRNA) | 2.46 × 10³ |

As can be seen in Table 5 above, it was confirmed that the use of LNPs including the ionizable lipid compound of the present invention showed an about 6,000- to 46,000-fold increase in the efficiency of mRNA delivery compared to when mRNA was used alone. Additionally, it was confirmed that the efficiency of mRNA delivery into cells was increased by about 10- to 80-fold when LNPs including the present ionizable lipid compound were used compared to when MC3 LNP was used.

### Experimental Example 3. Nucleic acid delivery efficacy of novel ionizable lipid compounds (in vivo)

In order to evaluate drug delivery distribution and *in vivo* transfection efficiency after systemic administration of the prepared substances, intravenous and intramuscular injections were separately evaluated.

### Experimental Example 3.1. Nucleic acid delivery efficacy following intravenous administration of novel ionizable lipid compounds

The LNP including MC3 (MC3:DSPC:Chol:DMG-PEG = 50:10:38.5:1.5) was used as a positive control group, PBS was used as a negative control group, and as the substances for experimental groups, the LNPs including Lipid 8-(F2), Lipid 9-(F2), Lipid 10-(F2), Lipid 11-(F2), or Lipid 13-(F2) shown in Table 3 were used.

For an intravenous administration group, LNPs corresponding to 0.5 mg/kg mRNA were injected into Balb/c mice (5-week-old male) via tail vein. Six hours after the injection, 150 mg/kg of D-Luciferin (Perkin Elmer, USA) was administered intraperitoneally, and 15 minutes thereafter, the luminescence intensity (total flux) in each mouse liver tissue was quantitatively analyzed using the IVIS imaging system Luminar XR (Perkin Elmer, USA), and the *in vivo* nucleic acid delivery efficacy of the novel ionizable lipid compounds was measured.

The quantified luminescence intensities in the experimental groups and control group are as shown in Table 6. Additionally, the relative luminescence intensities and the presence or absence of luminescence according to nucleic acid delivery in the experimental groups and control group are shown in Figs. 4 and 5, respectively.

**[Table 6]**

| | **Average Total Flux** |
|---|---|
| LNP including ionizable lipid compound 8 (Lipid 8-(F2)) | 2.53 × 10¹⁰ |
| LNP including ionizable lipid compound 9 (Lipid 9-(F2)) | 3.05 × 10¹⁰ |
| LNP including ionizable lipid compound 10 (Lipid 10-(F2)) | 7.08 × 10¹⁰ |
| LNP including ionizable lipid compound 11 (Lipid 11-(F2)) | 2.71 × 10¹⁰ |
| LNP including ionizable lipid compound 13 (Lipid 13-(F2)) | 2.39 × 10¹⁰ |
| MC3 LNP | 2.06 × 10¹⁰ |
| Control Group (PBS) | 5.89 × 10⁴ |

As can be seen in Table 6 above, the ionizable lipid compounds of the present invention showed superior *in vivo* mRNA delivery efficacy compared to the control group.

### Experimental Example 3.2. Nucleic acid delivery efficacy according to intramuscular administration of novel ionizable lipid compounds

The LNP including MC3 (MC3:DSPC:Chol:DMG-PEG = 50:10:38.5:1.5) was used as a positive control group, PBS was used as a negative control group, and as the substances for experimental groups, the LNPs including Lipid 3-(F1), Lipid 3-(F2), Lipid 8-(F1), Lipid 8-(F2), Lipid 9-(F1), Lipid 9-(F2), Lipid 10-(F1), Lipid 10-(F2), Lipid 11-(F1), Lipid 11-(F2), Lipid 12-(F1), or Lipid 13-(F1) shown in Table 3 were used.

As a group for intramuscular administration, lipid nanoparticles corresponding to 0.25 mg/kg mRNA were administered intramuscularly to the thigh muscles of Balb/c mice (5-week-old male). The mice in the control group were also administered with PBS into the thigh muscles in the same manner. Six hours after the injection, 150 mg/kg of D-Luciferin (Perkin Elmer, USA) was administered intraperitoneally, and 15 minutes thereafter, the luminescence intensity (total flux) in each mouse thigh muscle tissue was quantitatively analyzed using the IVIS imaging system Luminar XR (Perkin Elmer, USA), and the *in vivo* nucleic acid delivery efficacy of the novel ionizable lipid compounds was measured.

The quantified luminescence intensities in the experimental groups and control group are as shown in Table 7. Additionally, the relative luminescence intensities and the presence or absence of luminescence according to nucleic acid delivery in the experimental groups and control group are shown in Figs. 6 and 7, respectively.

**[Table 7]**

| | **Average Total Flux** |
|---|---|
| LNP including ionizable lipid compound 3 (Lipid 3-(F1)) | 3.31 × 10⁸ |
| LNP including ionizable lipid compound 3 (Lipid 3-(F2)) | 2.33 × 10⁸ |
| LNP including ionizable lipid compound 8 (Lipid 8-(F1)) | 1.47 × 10⁸ |
| LNP including ionizable lipid compound 8 (Lipid 8-(F2)) | 3.92 × 10⁸ |
| LNP including ionizable lipid compound 9 (Lipid 9-(F1)) | 7.10 × 10⁸ |
| LNP including ionizable lipid compound 9 (Lipid 9-(F2)) | 3.86 × 10⁸ |
| LNP including ionizable lipid compound 10 (Lipid 10-(F1)) | 3.88 × 10⁸ |
| LNP including ionizable lipid compound 10 (Lipid 10-(F2)) | 2.42 × 10⁸ |
| LNP including ionizable lipid compound 11 (Lipid 11-(F1)) | 1.83 × 10⁸ |
| LNP including ionizable lipid compound 11 (Lipid 11-(F2)) | 9.94 × 10⁸ |
| LNP including ionizable lipid compound 12 (Lipid 12-(F1)) | 1.95 × 10⁸ |
| LNP including ionizable lipid compound 13 (Lipid 13-(F1)) | 3.18 × 10⁸ |
| MC3 LNP | 3.31 × 10⁸ |
| Control Group (PBS) | 2.33 × 10⁸ |

As can be seen in Table 7 above, the LNPs including the ionizable lipid compounds of the present invention, regardless of the mixing ratio with lipids, showed significantly increased *in vivo* mRNA delivery efficacy compared to the control group.

## Claims

1. A compound represented by the following Formula (I) or a salt thereof:
L1-L2-L3-L4-L5 (I)
wherein L1 and L5 are each independently a group represented by following Formula (II), 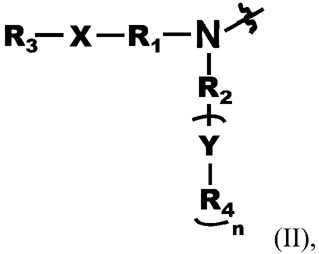
L2 is a C₁₋₁₂ linear alkyl, which is substituted or unsubstituted with a hydroxy group, in which when L2 is a C₁₋₁₂ linear alkyl substituted with a hydroxy group, Formula (I) does not include L1,
L4 is a C₁₋₁₂ linear alkyl,
L3 is a 6-membered heterocyclic amine or aliphatic amine, in which the 6-membered heterocyclic amine or aliphatic amine is substituted with at least one selected from the group consisting of C₁₋₆ alkyl, an amino group, 3- to 6-membered saturated or unsaturated carbocyclyl, 3- to 6-membered saturated or unsaturated carbocycloalkyl, 3-to 6-membered saturated or unsaturated heterocyclyl, and 3- to 6-membered saturated or unsaturated heterocycloalkyl,
n is an integer of 0 or 1,
X and Y are each independently -Rx'-C(=O)-O-, -Rx'-C(=O)-, -Rx'-NHC(=O)-, - C(=O)NH-Rx'-, -Rx'-O-, or -Rx'-NH-,
in which Rx' is hydrogen, linear C₁-₂₅ alkyl, or branched C₃-₂₅ alkyl,
R₁ and R₂, which are each independently linear C₁-₃₀ alkyl or branched C₃-₅₀ alkyl, are the same as or different from each other,
R₃ and R₄, which are each independently linear C₁-₃₀ alkyl, linear C₁-₃₀ alkylene, branched C₃-₅₀ alkyl or C₄₋₄₀ alkoxy, are the same as or different from each other, and the linear C₁-₃₀ alkyl, linear C₁-₃₀ alkylene, branched C₃-₅₀ alkyl, or C₄₋₄₀ alkoxy is substituted or unsubstituted with at least one selected from the group consisting of C₁₋₁₂ alkyl, Rx"-C(=O)-O-, Rx"-O-C(=O)-, and amine,
Rx" is hydrogen, linear C₁-₂₅ alkyl, branched C₃-₂₅ alkyl, 3- to 6-membered saturated or unsaturated carbocyclyl, 3- to 6-membered saturated or unsaturated carbocycloalkyl, 3-to 6-membered saturated or unsaturated heterocyclyl, or 3- to 6-membered saturated or unsaturated heterocycloalkyl,
the wavy line represents a bonding position,
the heterocyclic amine is a cyclic amine group including one or more heteroatoms selected from N, S, and O,
the carbocyclyl is a cyclic group consisting of carbon and hydrogen atoms,
the heterocyclyl is a cyclic group including one or more heteroatoms selected from N, S, and O,
the carbocycloalkyl is a carbocyclyl-linear C₁-₆ alkyl group or carbocyclyl-branched C₃₆ alkyl group, and
the heterocycloalkyl is a heterocyclyl-linear C₁-₆ alkyl group or heterocyclyl-branched C₃-₆ alkyl group.

2. The compound or the salt thereof according to claim 1, wherein L3 is a 6-membered heterocyclic amine.

3. The compound or the salt thereof according to claim 1 or 2, wherein L3 is a 6-membered heterocyclic amine including two nitrogen (N) atoms.

4. The compound or the salt thereof according to any one of claims 1 to 3, wherein L3 has a diazabicyclo structure.

5. The compound or the salt thereof according to any one of claims 1 to 4,
wherein L2 is linear C₁₋₆ alkyl or linear C₁₋₆ alkyl substituted with a hydroxy group, and wherein L4 is linear C₁₋₆ alkyl.

6. The compound or the salt thereof according to any one of claims 1 to 5,
wherein X and Y are each independently -Rx'-C(=O)-O-, -Rx'-C(=O)-, -Rx'-NHC(=O)-, -C(=O)NH-Rx'-, -Rx'-O-, or -Rx'-NH-, and
wherein Rx' is hydrogen, or linear C₁-₂₅ alkyl.

7. The compound or the salt thereof according to any one of claims 1 to 6, wherein R₁ and R₂ are each independently linear C₁-₃₀ alkyl, and R₁ and R₂ are the same as or different from each other.

8. The compound or the salt thereof according to any one of claims 1 to 7, wherein R₁ and R₂ are each independently linear C₁-₁₂ alkyl.

9. The compound or the salt thereof according to any one of claims 1 to 8, wherein R₃ and R₄ are each independently linear C₁-₂₀ alkyl, linear C₁-₂₀ alkylene, or branched C₃-₄₀ alkyl or C₄₋₃₀ alkoxy, and R₃ and R₄ are the same as or different from each other.

10. The compound or the salt thereof according to any one of claims 1 to 9, wherein
L2 is C₁₋₄ linear alkyl or C₁₋₄ linear alkyl substituted with a hydroxy group,
L3 has a diazabicyclo structure,
L4 is linear C₁₋₆ alkyl,
X and Y are each independently -Rx'-C(=O)-O-, -Rx'-C(=O)-, -Rx'-NHC(=O)-, - C(=O)NH-Rx'-, -Rx'-O-, or -Rx'-NH-,
Rx' is hydrogen or linear C₁-₁₂ alkyl,
R₁ and R₂ are each independently linear C₂-₈ alkyl and are the same as or different from each other, and
R₃ and R₄ are each independently linear C₁-₂₀ alkyl, linear C₁-₂₀ alkylene, or branched C₃-₄₀ alkyl or C₄₋₃₀ alkoxy and are the same as or different from each other, and the linear C₁-₂₀ alkyl, linear C₁-₂₀ alkylene, and branched C₃-₄₀ alkyl or C₄₋₃₀ alkoxy is unsubstituted or substituted with at least one selected from the group consisting of C₁₋₁₂ alkyl, Rx"-C(=O)-O-, Rx"-O-C(=O)-, and amine.

11. The compound or the salt thereof according to any one of claims 1 to 10,
wherein L1 and L5 are each independently a compound represented by the following Formula (III): 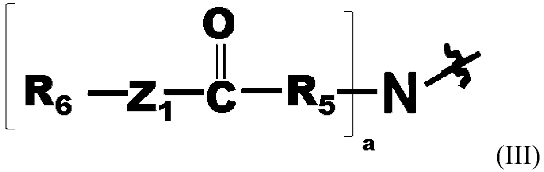
in which R₅ is linear C₁-₃₀ alkyl,
Z₁ is -(Rx^{a})ₖ-O- or -(Rx^{a})ₖ-NH-, Rx^{a} is linear C₁-₂₅ alkyl,
k is an integer of 0 or 1,
R₆ is linear C₁-₃₀ alkyl, linear C₁-₃₀ alkylene, branched C₃-₅₀ alkyl, or C₄₋₄₀ alkoxy, in which the linear C₁-₃₀ alkyl, linear C₁-₃₀ alkylene, branched C₃-₅₀ alkyl, or C₄₋₄₀ alkoxy is substituted or unsubstituted with at least one selected from the group consisting of C₁₋₁₂ alkyl, Rx^{b}-C(=O)-O-, Rx^{b}-O-C(=O)-, and amine,
Rx^{b} is hydrogen, linear C₁-₂₅ alkyl, branched C₃-₂₅ alkyl, 3- to 6-membered saturated or unsaturated carbocyclyl, 3- to 6-membered saturated or unsaturated carbocycloalkyl, 3-to 6-membered saturated or unsaturated heterocyclyl, or 3- to 6-membered saturated or unsaturated heterocycloalkyl,
a is an integer of 1 or 2,
the carbocyclyl is a cyclic group consisting of carbon and hydrogen atoms,
the heterocyclyl is a cyclic group including one or more heteroatoms selected from N, S, and O,
the carbocycloalkyl is a carbocyclyl-linear C₁-₆ alkyl group or carbocyclyl-branched C₃-₆ alkyl group, and
the heterocycloalkyl is a heterocyclyl-linear C₁-₆ alkyl group or heterocyclyl-branched C₃-₆ alkyl group.

12. The compound or the salt thereof according to claim 1, wherein the compound is a compound represented by the following Formula (IV): 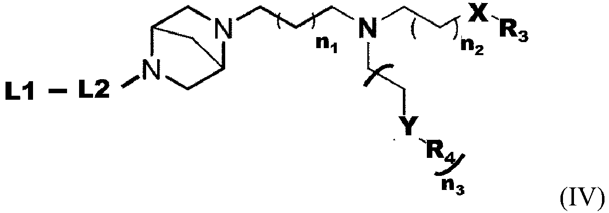
the definitions of L1, L2, X, Y, R₃, and R₄ are the same as those of L1, L2, X, Y, R₃, and
R₄ recited in claim 1,
n₁ and n₂ are each an integer of 0 to 12, and
n₃ is an integer of 0 or 1.

13. The compound or the salt thereof according to claim 1, wherein the compound is **characterized by** being selected from the group consisting of the followings:
(1) ((3,3',3",3‴-(((2,5-diazabicyclo[2.2.1]heptane-2,5-diyl)bis(propane-3,1-diyl))bis(azanetriyl)) tetrakis(propanoyl))tetrakis(oxy))tetrakis(butane-4,1-diyl)tetranonanoate;
(2) tetrakis(2-ethylhexyl) 3,3',3",3‴-(((2,5-diazabicyclo[2.2.1]heptane-2,5-diyl)bis(propane-3,1-diyl))bis(azanetriyl))tetrapropionate;
(3) tetrakis(4-(2-cyclohexylacetoxy)butyl) 3,3',3",3"'-(((2,5-diazabicyclo[2.2.1]heptane-2,5-diyl)bis(propane-3,1-diyl))bis(azanetriyl))tetrapropionate;
(4) ((3,3',3",3‴-(((2,5-diazabicyclo[2.2.1]heptane-2,5-diyl)bis(propane-3,1-diyl))bis(azanetriyl))tetrakis(propanoyl))tetrakis(oxy))tetrakis(butane-4,1-diyl)tetraheptanoate;
(5) ((3,3',3",3‴-(((2,5-diazabicyclo[2.2.1]heptane-2,5-diyl)bis(propane-3,1-diyl))bis(azanetriyl)) tetrakis(propanoyl))tetrakis(oxy))tetrakis(ethane-2,1-diyl)tetraheptanoate;
(6) ((3,3',3",3‴-(((2,5-diazabicyclo[2.2.1]heptane-2,5-diyl)bis(propane-3,1-diyl))bis(azanetriyl)) tetrakis(propanoyl))tetrakis(oxy))tetrakis(butane-4,1-diyl)tetratridecanoate;
(7) tetrakis(2-ethylhexyl) 4,4',4",4‴-((3,3',3",3‴-(((2,5-diazabicyclo[2.2.1]heptane-2,5-diyl)bis(propane-3,1-diyl))bis(azanetriyl))tetrakis(propanoyl))tetrakis(oxy))tetrabutyrate;
(8) tetrakis(2-ethylhexyl) 6,6',6",6‴-((3,3',3",3‴-(((2,5-diazabicyclo[2.2.1]heptane-2,5-diyl)bis(propane-3,1-diyl))bis(azanetriyl))tetrakis(propanoyl))tetrakis(oxy))tetrahexanoate;
(9) tetrakis(2-propylhexyl) 4,4',4",4‴-((3,3',3",3‴-(((2,5-diazabicyclo[2.2.1]heptane-2,5-diyl)bis(propane-3,1-diyl))bis(azanetriyl))tetrakis(propanoyl))tetrakis(oxy))tetrabutyrate;
(10) tetrakis(2-ethylpentyl) 4,4',4",4‴-((3,3',3",3‴-(((2,5-diazabicyclo[2.2.1]heptane-2,5-diyl)bis(propane-3,1-diyl))bis(azanetriyl))tetrakis(propanoyl))tetrakis(oxy))tetrabutyrate;
(11) tetrakis(2-ethylpentyl) 6,6',6",6‴-((3,3',3",3‴-(((2,5-diazabicyclo[2.2.1]heptane-2,5-diyl)bis(propane-3,1-diyl))bis(azanetriyl))tetrakis(propanoyl))tetrakis(oxy))tetrahexanoate;
(12) tetra((Z)-non-2-en-1-yl) 9,9',9",9‴-(((2,5-diazabicyclo[2.2.1]heptane-2,5-diyl)bis(propane-3,1-diyl))bis(azanetriyl))tetranonanoate;
(13) (((3,3',3",3‴-(((2,5-diazabicyclo[2.2.1]heptane-2,5-diyl)bis(propane-3,1-diyl))bis(azanetriyl))tetrakis(propanoyl))tetrakis(oxy))tetrakis(methylene))tetrakis(prop ane-2,1,3-triyl)octahexanoate;
(14) di(henicosan-10-yl) 3,3'-(((2,5-diazabicyclo[2.2.1]heptane-2,5-diyl)bis(propane-3,1-diyl))bis(methylazanediyl))dipropionate; and
(15) ((3,3'-((3-(5-(3-hydroxypropyl)-2,5-diazabicyclo[2.2.1]heptane-2-yl)propyl)azanediyl)bis (propanoyl))bis(oxy))bis(hexane-6,1-diyl)bis(2-hexyldecanoate).

14. A lipid nanoparticle comprising the compound or the salt thereof according to any one of claims 1 to 13, a helper lipid, cholesterol, and a PEG lipid.

15. The lipid nanoparticle according to claim 14, wherein the helper lipid is one or more phospholipids selected from the group consisting of dioleoylphosphatidylethanolamine (DOPE), distearoylphosphatidylcholine (DSPC), palmitoyloleoylphosphatidylcholine (POPC), 1-palmitoyl-2-oleoyl-sn-glycero-3-phosphocholine, egg phosphatidylcholine (EPC), dioleoylphosphatidylcholine (DOPC), dipalmitoylphosphatidylcholine (DPPC), dioleoylphosphatidylglycerol (DOPG), dipalmitoylphosphatidylglycerol (DPPG), distearoylphosphatidylethanolamine (DSPE), phosphatidylethanolamine (PE), dipalmitoylphosphatidylethanolamine (DPPE), 1-palmitoyl-2-oleoyl-sn-glycero-3-phosphoethanolamine (POPE), 1,2-dioleoyl-sn-glycero-3-[phospho-L-serine](DOPS), 1,2-dioctadecanoyl-sn-glycero-3-phosphoserine (DSPS), 1,2-di-(9Z-octadecenoyl)-sn-glycero-3-phospho-L-serine (DOPS) and sphingomyelin.

16. The lipid nanoparticle according to claim 14 or 15, wherein the PEG lipid includes a lipid directly conjugated to PEG or a lipid linked to PEG via a linker moiety,
wherein the linker moiety is one or more selected from the group consisting of carbonate (-OC(O)O-), succinoyl, phosphate ester (-O-(O)POH-O-), sulfonate ester, amido (-C(O)NH-), amino (-NR-), carbonyl (-C(O)-), carbamate (-NHC(O)O-), urea (-NHC(O)NH-), disulfide (-SS-), ether (-O-), succinyl (-(O)CCH₂CH₂C(O)-), and succinamidyl (-NHC(O)CH₂CH₂C(O)NH-),
the lipid linked to the PEG is ceramide, dimyristoylglycerol (DMG), succinoyl-diacylglycerol (s-DAG), distearoylphosphatidylcholine (DSPC), distearoylphosphatidylethanolamine (DSPE), or cholesterol.

17. The lipid nanoparticle according to any one of claims 14 to 16, wherein the lipid nanoparticle includes 20 to 80 mol% of the compound or the salt thereof, 0 to 20 mol% of the helper lipid, 20 to 65 mol% of the cholesterol, and 0 to 5 mol% of the PEG lipid, and wherein the helper lipid is phospholipid.

18. A composition including:
an ionic drug and
(i) the compound or the salt thereof of any one of claims 1 to 13, or (ii) a lipid nanoparticle including the compound or the salt thereof according to any one of claims 1 to 13, a helper lipid, cholesterol, and a PEG lipid.

19. The composition according to claim 18, wherein the ionic drug is one or more selected from the group consisting of nucleic acids, nucleic acid-based drugs, peptides, protein drugs, protein-nucleic acid constructs, and ionic biopolymer-drug conjugates.

20. The composition according to claim 19, wherein the nucleic acid is one or more selected from the group consisting of an antisense oligonucleotide (ASO), short interfering RNA (siRNA), micro RNA (miRNA), self-amplifying RNA (SAM), circular RNA, messenger RNA (mRNA), crRNA, tracrRNA, single guide RNA (sgRNA), transfer RNA (tRNA), asymmetric interference RNA (aiRNA), antagomir, ribozyme, dicer substrate RNA, short hairpin RNA (shRNA), plasmid DNA (pDNA), double stranded DNA (dsDNA), partial double stranded DNA, triple stranded DNA, partial triple stranded DNA, single stranded DNA (ssDNA), single stranded RNA (ssRNA), double stranded RNA (dsRNA), locked nucleic acid (LNA), a peptide nucleic acid (PNA), a miRNA analogue, and anti-miRNA.
